# EUROPEAN PATENT APPLICATION

(11) **EP 3 574 869 A1**
(43) Date of publication of application: **04.12.2019**
(21) Application number: 18744632.3
(22) Date of filing: 24.01.2018
(51) Int. Cl.: A61F 2/26

(54) **PENILE PACEMAKER AND CONTROL METHOD THEREOF**

(30) Priority: 24.01.2017 CN 201710054327
(71) Applicant: Shanghai Clinical Engine Technology Development Co., Ltd., Shanghai 200439 (CN)
(72) Inventor: SUN, Yinghao, Shanghai 200439 (CN)
(74) Representative: Viering, Jentschura & Partner mbB Patent- und Rechtsanwälte
(86) International application number: PCT/CN2018/073902
(87) International publication number: WO 2018/137632

(57) **Abstract**

A penis pacemaker and a method for controlling the same are disclosed. The penis pacemaker comprises a penis implanting mechanism (1), a reservoir (35), a catheter (2) and a pump (302), wherein the penis implanting mechanism (1) is arranged between a subcutaneous tissue and a cavernous body of the penis, comprises a hollow cavity and can be axially and elastically fixed at a proximal end of the penis; the reservoir (35) stores a fluid, and is communicated with the cavity of the penis implanting mechanism (1) via the at least one catheter (2); and the pump (302) is coupled to the reservoir (35) and the penis implanting mechanism (1), and used to provide power for the delivery of the fluid between the reservoir (35) and the penis implanting mechanism (1).

## Description

### TECHNICAL FIELD

The embodiments of the present invention relate to the technical field of medical apparatuses, in particular to a penis pacemaker and a method for controlling the same.

### BACKGROUND

For male patients with erectile dysfunction, the penis often cannot be erect autonomously or the erection hardness is poor. There is currently an implantable penile prosthesis on the market, as described in the patents US4651721 and US5062417, which is mainly composed of an implantable water bladder in the cavernous body, a fluid storage bladder and a one-way valve. Due to a complicated structure of the implanting device and large surgical trauma, especially the replacement of the cavernous body of the penis by the water bladder, the anatomical structure of the cavernous body itself is completely destroyed, accompanied with high surgical risk and long postoperative recovery time. Based on the above factors, the implantable penile prosthesis in the cavernous body is not an ideal auxiliary treatment device for erection.

As provided in the patent US20130131443, a skeleton prosthesis made of a temperature-sensitive shape memory alloy and wrapped outside the cavernous body of the penis also has large surgical trauma because the implantation cannot be completed until the cavernous body of the penis is completely dissociated at the time of implantation. In addition, since the prosthesis is made of a metal material, the foreign body sensation is noticeable and the use feeling is poor. The greatest disadvantage of this invention is that the temperature conditions for the deformation of the metal need to be provided for pacemaking and pacemaking stopping, which is very inconvenient to use. In view of the various problems existing in the prior art, there is an urgent need for a penile erection aid which is simple and minimally invasive in implantation manner, exact in auxiliary erection effect, and easy, safe and stable to use.

### SUMMARY

In view of the above problems, the present invention provides a penis pacemaker and a method for controlling the same to overcome the above problems or at least partially solve the above problems.

The penis implanting mechanism is arranged between a subcutaneous tissue and a cavernous body of the penis, comprises a hollow cavity and can be axially and elastically fixed at a proximal end of the penis;
the reservoir stores a fluid, and is communicated with the cavity of the penis implanting mechanism via the at least one catheter; and
the pump is coupled to the reservoir and the penis implanting mechanism; the pump is used to provide power for the delivery of the fluid between the reservoir and the penis implanting mechanism.

Optionally, in an embodiment of the present invention, the penis pacemaker further comprises a power source which is electrically connected to the pump and used to supply electric energy to the pump.

Optionally, in an embodiment of the present invention, the pump comprises a pump body, a pump head and a spring, wherein the pump head is connected with the pump body through the spring; the pump head adjusts a volume of the reservoir by means of a reciprocating motion; the spring at least partially drives the pump head to adjust the volume of the reservoir.

Optionally, in an embodiment of the present invention, the penis pacemaker further comprises a control device which is used to control the operation of the pump.

Optionally, in an embodiment of the present invention, the patient controls the control device in vitro.

Optionally, in an embodiment of the present invention, the control device comprises a limit switch which is connected in series with the pump and used to limit a travel of the pump.

Optionally, in an embodiment of the present invention, a valve is further arranged on the catheter and used to change a flowing direction of a fluid.

Optionally, in an embodiment of the present invention, at least one valve is further arranged on the catheter to acquire electric energy from the power source and change the flowing direction of the fluid under the control of the control device.

Optionally, in an embodiment of the present invention, the control device comprises an in-vitro wireless remote-control device and an in-vivo induction device, wherein the in-vivo induction device is used to be wirelessly coupled to the in-vitro wireless remote-control device and charge the power source according to a coupling signal.

Optionally, in an embodiment of the present invention, the in-vitro wireless remote-control device comprises a start button which is used to control the starting of the pump.

Optionally, in an embodiment of the present invention, at least one valve is further arranged on the catheter to acquire electric energy from the power source and change the flowing direction of the fluid under the control of the control device.

Optionally, in an embodiment of the present invention, a fixing device is arranged at a proximal end of the penis implanting mechanism and coupled to the pubis.

Preferably, the fixing device is at least one fixing plate provided with a through hole through which a fixing member fixes the fixing plate to the pubis.

Optionally, in an embodiment of the present invention, a base is arranged at the proximal end of the penis implanting mechanism; the fixing device is coupled to the base; the base is provided with a hollow cavity which is communicated with the cavity of the penis implanting mechanism.

Preferably, the shape of the base is matched with the shape of the cavernous body.

More preferably, the base comprises a raised upper surface and a recessed lower surface.

Optionally, in an embodiment of the present invention, the penis implementing mechanism comprises at least two segment-type structures of which the outer surfaces are elongated and retracted along an axial direction of the penis.

Optionally, in an embodiment of the present invention, the penis implanting mechanism comprises two segment-type structures, wherein end covers of the two segment-type structures are coupled with each other.

Preferably, sides of the two segment-type structures are coupled with each other.

Optionally, in an embodiment of the present invention, each segment-type structure comprises at least two and/or at least three corrugated tubes.

Preferably, each segment-type structure comprises at least three corrugated tubes.

Optionally, in an embodiment of the present invention, each segment-type structure comprises at least one outer-layer segment and at least one inner-layer segment, wherein the at least one outer-layer segment and the at least one inner-layer segment are nested with each other and are slidable relative to each other along an axial direction.

Preferably, a limiting device is arranged between each outer-layer segment and the corresponding inner-layer segment and used for preventing the outer-layer segment from slipping from the inner-layer segment.

Preferably, each of the at least one outer-layer segment and the at least one inner-layer segment comprises at least one first hardness segment and at least one second hardness segment, wherein the first hardness segments and the second hardness segments are distributed alternately; the hardness of the first hardness segment is greater than the hardness of the second hardness segment.

Optionally, in an embodiment of the present invention, each segment-type structure comprises one outer-layer segment at each of two ends thereof, wherein each of the outer-layer segments at two ends comprises a segment unit; each outer-layer segment and each inner-layer segment between two ends comprise two segment units respectively; each segment unit comprises two first hardness segments and one second hardness segment, wherein the second hardness segment is positioned between the two first hardness segments.

Optionally, in an embodiment of the present invention, each segment-type structure comprises at least one hollow variable-length structure and at least one hollow fixed-length structure, wherein the variable-length structure and the fixed-length structure are connected in series with each other.

Preferably, a supporting structure is arranged between the upper surface and the lower surface of the fixed-length structure and used for supporting the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, each segment-type structure comprises at least one flexible foldable structure; a folded portion of each flexible foldable structure is stretched during fluid injection to cause the penis implanting mechanism to elongate; when the fluid is discharged, the flexible foldable structure is folded into a predetermined shape by its own elastic force to cause the penile implant mechanism to retract.

Preferably, a supporting structure is respectively arranged between every two of the flexible foldable structures and used to support the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, when each segment-type structure is stretched, the segment-type structure is a closed structure composed of a raised outer surface and a recessed outer surface.

Preferably, the cross-section of the segment-type structure when stretched is in a crescent shape.

Optionally, in an embodiment of the present invention, a portion of each segment-type structure close to the distal end of the penis is sealed by an end cover, wherein the shape of the end cover is matched with the shape of the cavernous body.

Preferably, each end cover comprises a raised upper surface and a recessed lower surface.

Optionally, in an embodiment of the present invention, a base is arranged at the proximal end of the penis implanting mechanism; the fixing device is coupled to the base; the base is provided with a hollow cavity which is communicated with the cavity of the penis implanting mechanism.

Preferably, the shape of the base is matched with the shape of the cavernous body.

Preferably, the base comprises a raised upper surface and a recessed lower surface.

Optionally, in an embodiment of the present invention, the penis implanting mechanism has a thickness of 0.5mm to 5mm.

Preferably, the penis implanting mechanism has a thickness of 2mm to 3mm.

Optionally, in an embodiment of the present invention, the outer surface of the penis implant mechanism is provided with a coating layer having biocompatibility and ductility.

Optionally, in an embodiment of the present invention, the penis pacemaker further comprises a limit switch which is connected in series with the pump and used to limit a travel of the pump.

Optionally, in an embodiment of the present invention, a patient controls the control device in vitro.

Optionally, in an embodiment of the present invention, the control device comprises an in-vitro wireless remote-control device and an in-vivo induction device, wherein the in-vivo induction device is used to be wirelessly coupled to the in-vitro wireless remote-control device and control penile erection and weakness according to a coupling signal.

Optionally, in an embodiment of the present invention, the control device charges the power source according to the coupling signal, and the power source supplies electric energy to the pump.

Optionally, in an embodiment of the present invention, a valve is further arranged on the catheter and used to change a flowing direction of the fluid.

Optionally, in an embodiment of the present invention, the power source supplies electric energy to the valve, and the control device controls the valve to change a flowing direction of the fluid.

Optionally, in an embodiment of the present invention, a fixing device is arranged at the proximal end of the penis implanting mechanism and coupled to the pubis.

Optionally, in an embodiment of the present invention, the fixing device is at least one fixing plate provided with a through hole through which a fixing member fixes the fixing plate to the pubis.

Optionally, in an embodiment of the present invention, a base is arranged at the proximal end of the penis implanting mechanism; the fixing device is coupled to the base; the base is provided with a hollow cavity which is communicated with the cavity of the penis implanting mechanism.

Preferably, the shape of the base is matched with the shape of the cavernous body.

Preferably, the base comprises a raised upper surface and a recessed lower surface.

Optionally, in an embodiment of the present invention, the penis implementing mechanism comprises at least one segment-type structure of which the outer surface is elongated and retracted along an axial direction of the penis.

Optionally, in an embodiment of the present invention, the penis implanting mechanism comprises two segment-type structures, wherein end covers of the two segment-type structures are coupled with each other.

Preferably, sides of the two segment-type structures are coupled with each other.

Optionally, in an embodiment of the present invention, each segment-type structure comprises at least two corrugated tubes.

Preferably, each segment-type structure comprises at least three corrugated tubes.

Optionally, in an embodiment of the present invention, each segment-type structure comprises at least one outer-layer segment and at least one inner-layer segment, wherein the at least one outer-layer segment and the at least one inner-layer segment are nested with each other and are slidable relative to each other along an axial direction.

Preferably, a limiting device is arranged between each outer-layer segment and the corresponding inner-layer segment and used for preventing the outer-layer segment from slipping from the inner-layer segment.

Preferably, each of the at least one outer-layer segment and the at least one inner-layer segment comprises at least one first hardness segment and at least one second hardness segment, wherein the first hardness segments and the second hardness segments are distributed alternately; the hardness of the first hardness segment is greater than the hardness of the second hardness segment.

Optionally, in an embodiment of the present invention, each segment-type structure comprises one outer-layer segment at each of two ends thereof, wherein each of the outer-layer segments at two ends comprises a segment unit; each outer-layer segment and each inner-layer segment between two ends comprise two segment units respectively; and each segment unit comprises two first hardness segments and one second hardness segment, wherein the second hardness segment is positioned between the two first hardness segments.

Optionally, in an embodiment of the present invention, each segment-type structure comprises at least one hollow variable-length structure and at least one hollow fixed-length structure, wherein the variable-length structure and the fixed-length structure are connected in series with each other.

Preferably, a supporting structure is arranged between the upper surface and the lower surface of the fixed-length structure and used for supporting the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, each segment-type structure comprises at least one flexible foldable structure; a folded portion of each flexible foldable structure is stretched during fluid injection to cause the penis implanting mechanism to elongate; and when the fluid is discharged, the flexible foldable structure is folded into a predetermined shape by its own elastic force to cause the penile implant mechanism to retract.

Preferably, a supporting structure is respectively arranged between every two of the flexible foldable structures and used to support the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, when each segment-type structure is stretched, the segment-type structure is a closed structure composed of a raised outer surface and a recessed outer surface.

Preferably, the cross-section of each segment-type structure when stretched is in a crescent shape.

Optionally, in an embodiment of the present invention, a portion of each segment-type structure close to the distal end of the penis is sealed by an end cover, wherein the shape of the end cover is matched with the shape of the cavernous body.

Preferably, each end cover comprises a raised upper surface and a recessed lower surface.

Optionally, in an embodiment of the present invention, a base is arranged at the proximal end of the penis implanting mechanism; the fixing device is coupled to the base; the base is provided with a hollow cavity which is communicated with the cavity of the penis implanting mechanism.

Preferably, the shape of the base is matched with the shape of the cavernous body.

Preferably, the base comprises a raised upper surface and a recessed lower surface.

Optionally, in an embodiment of the present invention, the penis implanting mechanism has a thickness of 0.5mm to 5mm.

Preferably, the penis implanting mechanism has a thickness of 2mm to 3mm.

Optionally, in an embodiment of the present invention, the outer surface of the penis implanting mechanism is provided with a coating layer having biocompatibility and ductility.

Optionally, in an embodiment of the present invention, the pump delivers fluid in the reservoir to the penis implanting mechanism via a catheter under the driving of electrical energy, and delivers the fluid back to the reservoir via the catheter under the driving of mechanical energy. Correspondingly, an embodiment of the present invention provides a method for controlling a penis pacemaker comprising a penis implanting mechanism, a reservoir, a catheter and a pump; the penis implanting mechanism is arranged between a subcutaneous tissue and a cavernous body of the penis, wherein the penis implanting mechanism comprises a hollow cavity and can be axially and elastically fixed at a proximal end of the penis;
a fluid is stored in the reservoir, wherein the reservoir is communicated with the cavity of the penis implanting mechanism via the at least one catheter; and the reservoir is coupled to the pump and the penis implanting mechanism, wherein the pump is used to provide power for the delivery of the fluid between the reservoir and the penis implanting mechanism to cause penis erection or weakness;
wherein, the pump is used to provide power for the delivery of the fluid between the reservoir and the penis implanting mechanism to cause penis erection or weakness further comprising:
the pump drives the fluid to flow from the reservoir to the cavity of the penis implanting mechanism via the catheter, such that the penis implanting mechanism is axially elongated to cause the penis erection; and
the pump drives the fluid to flow from the cavity of the penis implanting mechanism to the reservoir via the catheter, such that the penis implanting mechanism is axially retracted to cause the penis weakness.

A further embodiment of the present invention provides a penis pacemaker, comprising a penis implanting mechanism, a reservoir, a catheter, a pump and a heating device, wherein

the penis implanting mechanism is arranged between a subcutaneous tissue and a cavernous body of the penis, comprises a hollow cavity and can be axially and elastically fixed at a proximal end of the penis;
the reservoir stores a fluid, and is communicated with the cavity of the penis implanting mechanism via the at least one catheter;
the pump is coupled to the reservoir and the penis implanting mechanism; the pump is used to provide power for the delivery of the fluid between the reservoir and the penis implanting mechanism; and
the heating device is coupled to the reservoir and used to heat the fluid outputted from the reservoir to the penis implanting mechanism.

Optionally, in an embodiment of the present invention, the penis pacemaker further comprises a power source, wherein the power source is electrically connected to the pump and/or the heating device and used to supply electric energy to the pump and/or the heating device.

Preferably, the penis pacemaker further comprises a power source which is electrically connected to the pump and the heating device and used to supply electric energy to the pump and the heating device.

Optionally, in the embodiment of the present invention, the penis pacemaker further comprises a control device which is used to control the operation of the pump and/or the operation of the heating device.

Preferably, the control device is used to control the operation of the pump and the operation of the heating device.

Optionally, in an embodiment of the present invention, the penis pacemaker further comprises a temperature sensor, wherein the temperature sensor is coupled to the heating device and used to detect a temperature of the fluid heated by the heating device in real time; the control device controls the heating device to stop heating according to the temperature detected by the temperature sensor.

Optionally, in an embodiment of the present invention, the pump delivers fluid in the reservoir to the penis implanting mechanism via a catheter under the driving of electrical energy, and delivers the fluid back to the reservoir via the catheter under the driving of mechanical energy.

Preferably, the pump comprises a pump body, a pump head and a spring, wherein the pump head is connected with the pump body through the spring; the pump head adjusts a volume of the reservoir by means of a reciprocating motion; the spring at least partially drives the pump head to adjust the volume of the reservoir.

Optionally, in an embodiment of the present invention, the control device is further used to control the heating device, and control the heating device and the pump independent of each other.

Optionally, in an embodiment of the present invention, a time-delay circuit is coupled between the heating device and the pump, wherein the time-delay circuit is used to control a starting time of the heating device to be earlier than a starting time of the pump.

Preferably, at least one capacitor is arranged between the heating device and the pump; the heating device is connected in series with the pump; at least one capacitor is connected in parallel to the pump.

Optionally, in an embodiment of the present invention, the patient controls the control device in vitro.

Optionally, in an embodiment of the present invention, the control device comprises an in-vitro wireless remote-control device and an in-vivo induction device, wherein the in-vivo induction device is used to be wirelessly coupled to the in-vitro wireless remote-control device and charge the power source according to a coupling signal.

Optionally, in an embodiment of the present invention, the control device charges the power source according to the coupling signal, and the power source supplies electric energy to the pump.

Optionally, in an embodiment of the present invention, the control device is further used to control the heating device, and control the heating device and the pump simultaneously.

Optionally, in an embodiment of the present invention, a time-delay circuit is coupled between the heating device and the pump, wherein the time-delay circuit is used to control a starting time of the heating device to be earlier than a starting time of the pump.

Preferably, at least one capacitor is arranged between the heating device and the pump; the heating device is connected in series with the pump; at least one capacitor is connected in parallel to the pump.

Optionally, in an embodiment of the present invention, the penis pacemaker further comprises a limit switch which is connected in series with the pump and used to limit a travel of the pump.

Optionally, in an embodiment of the present invention, the control device comprises an in-vitro wireless remote-control device and an in-vivo induction device, wherein the in-vivo induction device is used to be wirelessly coupled to the in-vitro wireless remote-control device and charge the power source according to a coupling signal.

Optionally, in an embodiment of the present invention, the in-vitro wireless remote-control device comprises a start button which is used to control the starting of the pump and/or the starting of the heating device.

Preferably, the in-vitro wireless remote-control device comprises a start button which is used to control the pump and the heating device simultaneously, and a starting time of the heating device is earlier than a starting time of the pump.

Optionally, in an embodiment of the present embodiment, the penis pacemaker further comprises a limit switch which is connected in series with the pump and used to limit a travel of the pump.

At least one valve is further arranged on the catheter to acquire electric energy from the power source and change the flowing direction of the fluid under the control of the control device.

Optionally, in an embodiment of the present invention, the heating device is arranged on the upper surface inside the reservoir.

Optionally, in an embodiment of the present invention, the penis pacemaker further comprises a temperature sensor, wherein the temperature sensor is coupled to the heating device and used to detect a temperature of the fluid heated by the heating device in real time; the control device controls the heating device to stop heating according to the temperature detected by the temperature sensor.

Optionally, in an embodiment of the present invention, the temperature sensor has a threshold; when a temperature detected by the temperature sensor reaches the threshold, the heating device is turned off; the threshold is 34°C to 38°C.

Preferably, in an embodiment of the present invention, the threshold is 36.5°C to 37.5°C.

Optionally, in an embodiment of the present invention, the patient controls the control device in vitro.

Optionally, in an embodiment of the present invention, at least one valve is further arranged on the catheter, wherein the valve acquires electrical energy from the power source and changes a flowing direction of the fluid under the control of the control device.

Optionally, in an embodiment of the present invention, a fixing device is arranged at the proximal end of the penis implementing mechanism and coupled to the pubis.

Optionally, in an embodiment of the present invention, the fixing device is at least one fixing plate provided with a through hole through which a fixing member fixes the fixing plate to the pubis.

Optionally, in an embodiment of the present invention, a base is arranged at the proximal end of the penis implanting mechanism; the fixing device is coupled to the base; the base is provided with a hollow cavity which is communicated with the cavity of the penis implanting mechanism.

Preferably, the shape of the base is matched with the shape of the cavernous body.

Preferably, the base comprises a raised upper surface and a recessed lower surface.

Optionally, in an embodiment of the present invention, the penis implementing mechanism comprises at least one segment-type structure of which the outer surface is elongated and retracted along an axial direction of the penis.

Optionally, in an embodiment of the present invention, the penis implanting mechanism comprises two segment-type structures, wherein end covers of the two segment-type structures are coupled with each other.

Preferably, sides of the two segment-type structures are coupled with each other.

Optionally, in an embodiment of the present invention, each segment-type structure comprises at least two and/or at least three corrugated tubes.

Preferably, each segment-type structure comprises at least three corrugated tubes.

Optionally, in an embodiment of the present invention, each segment-type structure comprises at least one outer-layer segment and at least one inner-layer segment, wherein the at least one outer-layer segment and the at least one inner-layer segment are nested with each other and are slidable relative to each other along an axial direction.

Preferably, a limiting device is arranged between each outer-layer segment and the corresponding inner-layer segment and used for preventing the outer-layer segment from slipping from the inner-layer segment.

Preferably, each of the at least one outer-layer segment and the at least one inner-layer segment comprises at least one first hardness segment and at least one second hardness segment, wherein the first hardness segments and the second hardness segments are distributed alternately; the hardness of the first hardness segment is greater than the hardness of the second hardness segment.

Optionally, in an embodiment of the present invention, each segment-type structure comprises one outer-layer segment at each of two ends thereof, wherein each of the outer-layer segments at two ends comprises a segment unit; each outer-layer segment and each inner-layer segment between two ends comprise two segment units respectively; each segment unit comprises two first hardness segments and one second hardness segment, wherein the second hardness segment is positioned between the two first hardness segments.

Optionally, in an embodiment of the present invention, each segment-type structure comprises at least one hollow variable-length structure and at least one hollow fixed-length structure, wherein the variable-length structure and the fixed-length structure are connected in series with each other.

Preferably, a supporting structure is arranged between the upper surface and the lower surface of the fixed-length structure and used for supporting the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, each segment-type structure comprises at least one flexible foldable structure; a folded portion of each flexible foldable structure is stretched during fluid injection to cause the penis implanting mechanism to elongate; when the fluid is discharged, the flexible foldable structure is folded into a predetermined shape by its own elastic force to cause the penile implant mechanism to retract.

Preferably, a supporting structure is respectively arranged between every two of the flexible foldable structures and used to support the penis implanting mechanism in a circumferential direction and an axial direction.

Preferably, each supporting structure comprises an integrated pulling structure, wherein the integrated pulling structures are distributed in the circumferential direction and the axial direction of the segment-type structures.

Optionally, in an embodiment of the present invention, when each segment-type structure is stretched, the segment-type structure is a closed structure composed of a raised outer surface and a recessed outer surface.

Preferably, the cross-section of each segment-type structure when stretched is in a crescent shape.

Optionally, in an embodiment of the present invention, a portion of each segment-type structure close to the distal end of the penis is sealed by an end cover, wherein the shape of the end cover is matched with the shape of the cavernous body.

Preferably, each end cover comprises a raised upper surface and a recessed lower surface.

Optionally, in an embodiment of the present invention, a base is arranged at the proximal end of the penis implanting mechanism; the fixing device is coupled to the base; and the base is provided with a hollow cavity which is communicated with the cavity of the penis implanting mechanism.

Preferably, the shape of the base is matched with the shape of the cavernous body.

Preferably, the base comprises a raised upper surface and a recessed lower surface.

Optionally, in an embodiment of the present invention, the penis implanting mechanism has a thickness of 0.5mm to 5mm.

Preferably, the penis implanting mechanism has a thickness of 2mm to 3mm.

Optionally, in an embodiment of the present invention, the outer surface of the penis implanting mechanism is provided with a coating layer having biocompatibility and ductility.

Correspondingly, an embodiment of the present invention provides a method for controlling a penis pacemaker comprising a penis implanting mechanism, a reservoir, a catheter, a pump and a heating device, the penis implanting mechanism is arranged between a subcutaneous tissue and a cavernous body of the penis, wherein the penis implanting mechanism comprises a hollow cavity and can be axially and elastically fixed at a proximal end of the penis;
a fluid is stored in the reservoir, wherein the reservoir is communicated with the cavity of the penis implanting mechanism via the at least one catheter; and the reservoir is coupled to the pump and the penis implanting mechanism, wherein the pump is used to provide power for the delivery of the fluid between the reservoir and the penis implanting mechanism to cause penis erection or weakness;
the heating device is coupled to the reservoir and used to heat the fluid outputted from the reservoir to the penis implanting mechanism.
wherein, the pump is used to provide power for the delivery of the fluid between the reservoir and the penis implanting mechanism to cause penis erection or weakness further comprising:
the pump drives the fluid to flow from the reservoir to the cavity of the penis implanting mechanism via the catheter, such that the penis implanting mechanism is axially elongated to cause the penis erection; and
the pump drives the fluid to flow from the cavity of the penis implanting mechanism to the reservoir via the catheter, such that the penis implanting mechanism is axially retracted to cause the penis weakness.

As can be seen from the above technical solutions, the embodiments of the present invention have the following advantages.

The traditional penis implanting mechanism consists of two water bladders which are directly implanted into the two cavernous bodies, but the implantation cannot be completed until the cavernous body tissues are completely dissociated respectively during a surgical procedure, such that the anatomical structure of the cavernous tissues themselves is destroyed and a patient may even permanently lose the possibility of recovery. However, the penis implanting mechanism in the embodiment of the present invention is arranged between the subcutaneous tissue and the cavernous body of the penis, and has a thickness far smaller than the diameter or the thickness of the traditional water bladder, thereby hardly damaging the anatomical structure of the cavernous body tissues.

The ends of the water bladders of the traditional penis implanting mechanism are inserted into the body through two tapered structures. However, the penis implanting mechanism in the embodiment of the present invention is arranged between the subcutaneous tissue and the cavernous body of the penis, and is thus not suitable for being inserted into the body in a tapered structure, instead being fixed to the pubis by a fixing device such as a fixing plate. Therefore, the penis implanting mechanism can be fixed more firmly, and is not prone to shifting.

Further, in the embodiment of the present invention, the outer surface of the penis implanting mechanism is designed as segment-type structures that can be elongated and retracted along the axial direction of the penis, for example, in a manner of a plurality of corrugated tubes structures, mutual nesting of segment-type hollow structures of different hardness, or mutually serial connection of variable-length structures and fixed-length structures. Therefore, when the penis is in an erection, the penis implanting mechanism can be elongated, which causes the penis to elongate. When the penis is weak, the penile implanting mechanism retracts to a length consistent with the length of the penis.

In addition, the hollow base is arranged at the proximal end of each segment-type structure, and the cavity of the base is communicated with the cavity of the segment-type structure, such that the segment-type structure can be firmly fixed between the subcutaneous tissue and the cavernous body of the penis by the base, without shifting. On the other hand, when a plurality of segment-type structures is arranged, after the pump is started, the fluid is first outputted to the bases and then evenly distributed in the segment-type structures via the bases. The bases simultaneously function to secure the penis implanting mechanism and distribute the fluid evenly.

In an embodiment of the present invention, the heating device is additionally arranged in the penis pacemaker to heat the fluid outputted from the reservoir to the penis implanting mechanism, thereby eliminating the temperature difference between the fluid in the reservoir and the penis part, such that a patient feels more comfortable.

Furthermore, the temperature sensor is further additionally arranged in the penis pacemaker. The heating device is powered by the power source. The control device controls the heating device and the temperature sensor. The temperature sensor has a threshold. After the temperature of the fluid reaches the threshold, a signal is transmitted to the control device. The control device then controls the pump to deliver the fluid from the reservoir to the penis implanting mechanism, thereby effectively controlling the heating temperature of the fluid.

In a further embodiment of the present invention, the heating device and the temperature sensor are controlled in a remotely controlled manner, such that the patient's operation is more convenient. Further, the time-delay circuit is arranged between the heating device and the pump. For example, the starting time of the heating device is caused to be earlier than the starting time of the pump by means of connecting the capacitor in parallel with the pump, etc. Therefore, the fluid in the reservoir is prevented from flowing into the penis implanting mechanism without being heated, and meanwhile the structure of a remote-controller can also be simplified, for example, buttons that control the heating device and the pump can be combined into one.

### BRIEF DESCRIPTION OF THE DRAWINGS

In order to more clearly illustrate the embodiments of the present invention or the technical solutions in the prior art, the drawings used in the embodiments or the prior art description will be briefly described below. It is apparent that the drawings in the following description are only some of the embodiments described in the embodiments of the present invention, and those ordinary skilled in the art may also obtain other drawings based on these drawings.
Figure 1 is a schematic diagram of a penis pacemaker having a water bladder structure in the prior art.
Figures 2A-2E are schematic diagrams of five embodiments of a penis pacemaker of the present invention.
Figure 3 is a schematic diagram of a preferred embodiment based on Figure 2B-2E.
Figure 4 is a schematic diagram of an embodiment of an in-vitro charging control device and an in-vivo induction device based on FIG. 2C and Figure 2E.
Figure 5 is a schematic diagram of a preferred embodiment of the penis pacemaker of the present invention based on Figure 2E.
Figure 6 is a schematic diagram of an embodiment of a control circuit of the penis pacemaker of the present invention based on Figure 5.
Figure 7 is a schematic diagram of a preferred embodiment of a pump of the penis pacemaker of the present invention based on Figures 2D-2E.
Figure 8 is a schematic diagram of an embodiment in which a penis implanting mechanism is fixed to a proximal end of the penis based on Figures 2A-2E.
Figures 9A-9D are schematic diagrams of an embodiment of the penis implanting mechanism based on Figures 2A-2E.
Figure 10A is a top view of an embodiment of a component of the penis implanting mechanism based on Figure 9A.
Figure 10B is a schematic diagram of a cross-section of the penis implanting mechanism perpendicular to an axial direction based on Figure 9A.
Figure 11A is a schematic diagram of a cross-section of the penis implanting mechanism perpendicular to an axial direction based on Figure 9B.
Figure 11b is a locally enlarged view of a cross-section of the penis implanting mechanism along an axial direction based on Figure 9B.
Figure 12 is a schematic diagram of a cross-section of the penis implanting mechanism perpendicular to an axial direction based on Figure 9C.
Figure 13A is a schematic diagram of a cross-section of the penis implanting mechanism perpendicular to an axial direction based on Figure 9D.
Figure 13B is a locally enlarged view of a retracted segment-type structure of the embodiment of the penis implanting mechanism based on Figure 9D.
Figures 14A-14B are integral schematic diagrams of the penis implanting mechanism of the penis pacemaker of the present invention in the penis.
Figure 15 is a schematic diagram of a cross-section of the penis implanting mechanism of the penis pacemaker of the present invention in the penis.

### DETAILED DESCRIPTION OF THE INVENTION

Of course, it is unnecessary to achieve all the above advantages at the same time to implement any technical solution of the embodiments of the present invention.

For a better understanding of the technical solutions in the embodiments of the present invention, the technical solutions in the embodiments of the present invention are clearly and completely described below with reference to the accompanying drawings in the embodiments of the present invention. It is apparent that the described embodiments are only a part of the embodiments of the present invention, and not all of the embodiments. All other embodiments obtained by those ordinary skilled in the art based on the embodiments of the present invention should fall within the scope of protection of the embodiments of the present invention.

The "distal end of the penis" refers to the end of the penis away from the human body. The "proximal end of the penis" refers to the end of the penis close to the human body. The "segment-type structure" means a structure composed of at least one section or segment. The "proximal end of the penis implanting mechanism" refers to the end of the penis implanting mechanism close to the human body. The "proximal end of the segment-type structure" refers to the end of the segment-type structure close to the human body. The "axial direction" refers to a direction extending along the length of the penis. The "thickness of the penis implanting mechanism" refers to a maximum distance between an upper surface and a lower surface of the penis implanting mechanism when the penis is in erection. The "fluid" includes, but is not limited to, a liquid, a gas, such as a disinfectant, physiological saline, or air.

The specific implementations of the embodiments of the present invention are further described below in conjunction with the accompanying drawings.

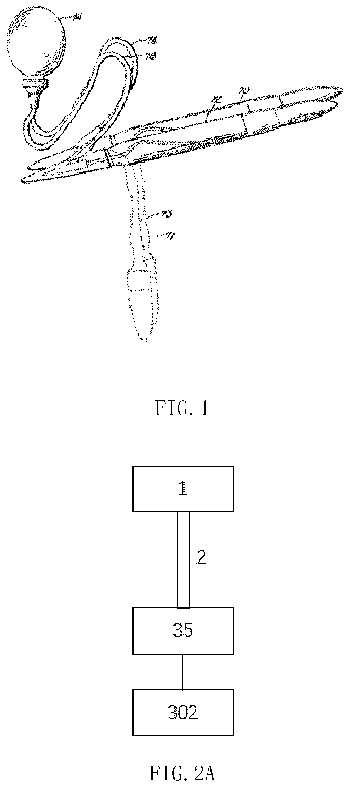

Figure 1 is a penis pacemaker having a water bladder structure in the prior art. This penis pacemaker consists of two water bladders. A radial diameter of each water bladder ranges from 8mm to 10mm. The water bladders are implanted to two cavernous bodies directly. The implantation cannot be completed until cavernous body tissues are completely dissociated respectively during a surgical procedure, such that the anatomical structure of the cavernous tissues themselves is destroyed and a patient may even permanently lose the possibility of recovery.

Figures 2A-2E show five embodiments of a penis pacemaker of the present invention.

Figure 2A shows a manual, non-electric penis pacemaker, comprising a penis implanting mechanism 1, a catheter 2, a reservoir 35, and a pump 302. The penis implanting mechanism 1 is arranged between a subcutaneous tissue and a cavernous body of the penis, comprises a hollow cavity and can be axially and elastically fixed at a proximal end of the penis. The reservoir 35 stores a fluid, and is communicated with a cavity of the penis implanting mechanism 1 via the at least one catheter 2. The pump 302 is coupled to the reservoir 35 and the penis implanting mechanism 1. The pump 302 is used to deliver the fluid between the reservoir 35 and the penis implanting mechanism 1. The reservoir 5 stores a predetermined volume of fluid. The volume of the fluid is preferred to at least completely fill the cavity of the penis implanting mechanism 1.

The patient squeezes the pump 302 by hand such that the pump 302 pushes the fluid in the reservoir 35 and the fluid flows into the cavity of the penis implanting mechanism 1 through the catheter 2, causing the penis implanting mechanism 1 elongate, or manually controls the fluid to flow back to the reservoir 35 from the cavity of the penis implanting mechanism 1, causing the penis implanting mechanism 1 to retract. Preferably, in an embodiment of the present invention, the catheter is further provided with a valve used to change a flowing direction of the fluid.

The catheter 2 is a hollow catheter of which a wall lining can have a strengthened structure, such as a strengthened mesh-like woven structure. One or more catheters 2 may be arranged.

The reservoir 35 and the pump 302 may be two independent devices, or may be of an integrated structure. The pump may be a one-way pump or a two-way pump.

The following is an embodiment in which the reservoir 35 and the pump 302 are two independent devices:

the pump 302 is located between the reservoir 35 and the penis implanting mechanism 1; the pump is connected to the reservoir 35 and the penis implanting mechanism 1 respectively via the catheter; the pump 302 may be a one-way pump or a two-way pump. The pump 302 pumps the fluid out of the reservoir 35 and re-delivers the fluid to the penis implanting mechanism 1 to cause the penis to erect. When the penis requires weakness, the pump 302 pumps the fluid back from the penis implanting mechanism 1 and re-delivers it back into the reservoir 35. A valve may be arranged between the pump and each of the reservoir 35 and the penis implanting mechanism 1 to control the flow of fluid.

The following is an embodiment in which the reservoir 35 and the pump 302 are of an integrated structure:

the pump 302 and the reservoir 35 are integrated into a whole and are separated by a diaphragm, a piston or other appropriate structure. When the pump 302 is started, the pump 302 extrudes the reservoir 35, such that the volume of the reservoir 35 reduces, and the fluid flows out of the reservoir 35.

The reservoir 35 and the pump 302 are individually (independently mounted) or collectively (integrally constructed) covered by a housing 31. The housing 31 is made of a biocompatible material, such as stainless steel, titanium alloy, cobalt chromium alloy, or the like. In addition, the surface of the housing may be sprayed or plated with a material, such as parylene, Teflon, polysaccharide polymer, or modified silicone, for improving the surface properties of the housing and enhancing the biocompatibility and stability.

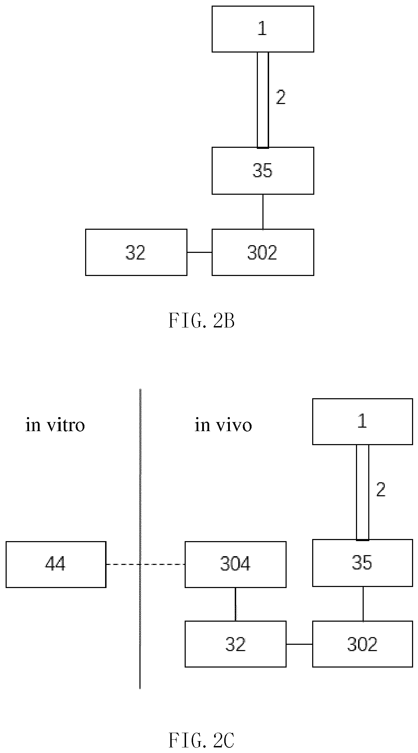

Figure 2B is a manual, electric penis pacemaker. Compared with Figure 2A, a power source 32 is additionally arranged in Figure 2B. The power source 32 is electrically connected to the pump 302, and used to supply electric energy to the pump 302. The power source 32 is a battery or other device capable of supplying electric energy.

The power source 32 may be separated from the reservoir 35 and the pump 302, or be integrated with the pump 302, or be integrated with the reservoir 35 and the pump 302. The power source 32 may be arranged in the scrotum or other parts of the human body together with the reservoir 35 and the pump 302.

Preferably, the penis pacemaker further comprises a control device 702. The control device is used to control the operation of the pump and/or the operation of the heating device. Preferably, the control device is used to control the operation of the pump and the operation of the heating device. Optionally, in the embodiment of the present invention, the patient controls the control device 702 in vitro.

In an embodiment of the present invention, the control device 702 is located in the scrotum. The power source 32 supplies, for example, electric energy to the pump 302 when the patient turns on a switch in vitro by applying an external force to the scrotum. The pump 302 is started to push the fluid in the reservoir 35, such that the fluid flows into the cavity of the penis implanting mechanism 1 through the catheter 2, causing the penis implanting mechanism 1 to elongate. When the switch is turned off, the fluid flows from the cavity of the penis implanting mechanism 1 back to the reservoir 35, causing the penis implanting mechanism 1 to retract. Preferably, the fluid in the reservoir 35 just satisfies the full elongation of the penis implanting mechanism 1. When the fluid completely flows into the penis implanting mechanism 1, the switch is turned off and the pump 302 is stopped.

Preferably, the pump 302 may also comprise a motor capable of converting electric energy into a mechanical motion, or a power system consisting of a motor and a transmission mechanism. The motor may be a rotating motor or a linear motor. The transmission mechanism may be a common transmission mechanism such as a gear, a turbine, or a ball screw.

Optionally, in an embodiment of the present invention, the pump delivers the fluid in the reservoir to the penis implanting mechanism via the catheter under the driving of electric energy, and delivers the fluid back to the reservoir via the catheter under the driving of mechanical energy.

Preferably, the pump comprises a pump body, a pump head and a spring. The pump head is connected with the pump body through the spring. The pump head adjusts a volume of the reservoir by means of a reciprocating motion. The spring at least partially drives the pump head to adjust the volume of the reservoir.

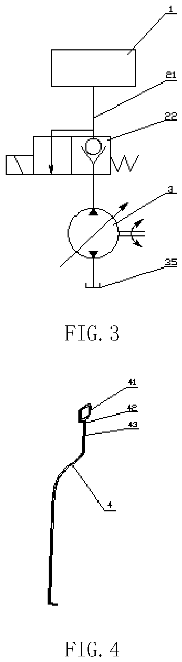

Figure 3 is a schematic diagram of a preferred embodiment based on Figures 2B-2D. A valve 22 is further arranged on the catheter 2 and is electrically connected to the power source 32. The valve acquires electric energy from the power source and changes a flowing direction of the fluid under the control of the control device. The valve 22 is an electromagnetic valve or other valve 22 that is controllable by electrical energy. For example, when the pump 302 pumps fluid from the reservoir 35 into the penis implanting mechanism 1, the valve 22 can be automatically opened under the action of hydraulic pressure. When the fluid flows from the penis implanting mechanism 1 back to the reservoir 35, the valve 22 is energized, and the control device 702 controls the valve 22 to be opened to complete the drainage of the penis implanting mechanism 1.

The valve 22 may be located at one end of the catheter 2 close to the penis implanting mechanism 1 or at one end of the catheter 2 close to the reservoir 35. Although the valve 22 is arranged here on the catheter 2, the valve 22 may still be arranged in the penis implanting mechanism 1. The valve 22 can be a valve or a plurality of valves. In an embodiment of the present invention, when one catheter 2 is arranged, one valve 22 is correspondingly provided. When two catheters are arranged, a one-way valve is correspondingly arranged on each catheter, or a common reversing valve is correspondingly arranged on the two catheters.

Figure 2C is a remotely-controlled, electric penis pacemaker. The control device 702 comprises an in-vitro wireless remote-control device 44 and an in-vivo induction device 304. The in-vivo induction device 304 is used to be wirelessly coupled to the in-vitro wireless remote-control device 44 and control penile erection and weakness according to a coupling signal.

Optionally, in an embodiment of the present invention, the control device 702 charges the power source according to the coupling signal. The power source supplies electric energy to the pump. The power source 32 is a rechargeable battery, and may be a storage battery or other rechargeable battery. Types of the storage batteries include a lithium ion battery, a lithium-iodine battery, and a graphene battery.

Preferably, the in-vitro wireless remote-control device 44 is correspondingly provided with at least one button for controlling the in-vitro wireless remote-control device 44 to send a signal. The in-vivo induction device 304 receives the signal and converts the signal into a current to charge the power source. The structure of the in-vitro wireless remote-control device 44 is the structure of the existing remote-controller, and will not be described herein again.

Preferably, the in-vitro wireless remote-control device 44 comprises a signal transmitting antenna. The in-vivo induction device 304 includes a receiving antenna. The receiving antenna may be implanted into the patient's body, for example, abdominally or subcutaneously. When the receiving antenna is implanted subcutaneously, a coil of the antenna may be smaller than a coil of the receiving antenna that is implanted abdominally.

Optionally, in an embodiment of the present invention, a valve 22 is further arranged on the catheter 2 and is electrically connected to the power source 32. The valve acquires electric energy from the power source and changes a flowing direction of the fluid under the control of the control device. The valve 22 is an electromagnetic valve or other valve 22 that is controllable by electrical energy. The valve 22 may be controlled by the in-vitro wireless remote-control device 44.

In a further embodiment of the present invention, the control device 702 is provided with a fluid flow detection unit in which a fluid flow threshold is further stored. When the flow of fluid from the reservoir 35 into the penis implanting mechanism 1 or from the penis implanting mechanism 1 back to the reservoir 35 reaches a threshold, the valve 22 is automatically closed. The threshold is preferably the volume of fluid just filling the penis implant mechanism 1.

In a further embodiment of the present invention, the control device 702 is not provided with a fluid flow detection unit. The patient directly controls the valve 22 to be opened or closed via the control device 702 by virtue of experiences.

Optionally, in an embodiment of the present invention, the pump delivers the fluid in the reservoir to the penis implanting mechanism via the catheter under the driving of electric energy, and delivers the fluid back to the reservoir via the catheter under the driving of mechanical energy.

Preferably, the pump comprises a pump body, a pump head and a spring. The pump head is connected with the pump body through the spring. The pump head adjusts a volume of the reservoir by means of a reciprocating motion. The spring at least partially drives the pump head to adjust the volume of the reservoir.

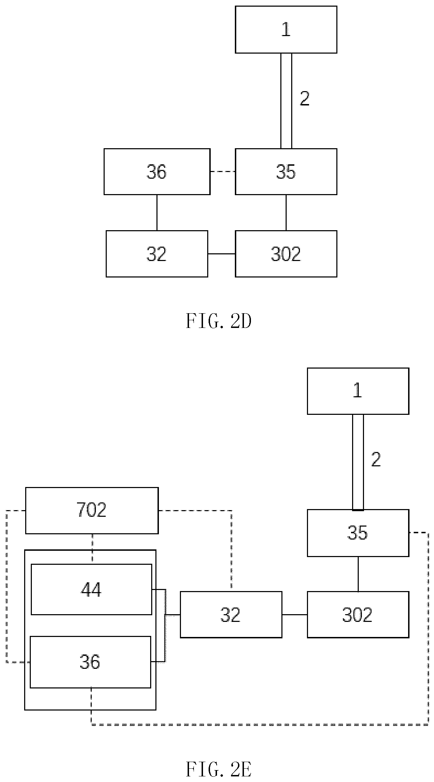

Figure 2D is a heating type, electric penis pacemaker, comprising a penis implanting mechanism 1, a reservoir 35, a catheter 2, a pump 302 and a heating device 36. The penis implanting mechanism 1 is arranged between a subcutaneous tissue and a cavernous body of the penis, comprises a hollow cavity and can be axially and elastically fixed at a proximal end of the penis. The reservoir 35 stores a fluid, and is communicated with the cavity of the penis implanting mechanism 1 via the at least one catheter 2.

The pump 302 is coupled to the reservoir 35 and the penis implanting mechanism 1. The pump 302 is used to provide power for the delivery of the fluid between the reservoir 35 and the penis implanting mechanism 1.

The heating device 36 is coupled to the reservoir 35 and used to heat the fluid outputted from the reservoir 35 to the penis implanting mechanism 1.

Optionally, in an embodiment of the present invention, the penis pacemaker further comprises a power source 32. The power source 32 is electrically connected to the pump 302 and/or the heating device and used to supply electric energy to the pump 302 and/or the heating device.

Optionally, in an embodiment of the present invention, the power source supplies electric energy to the heating device.

Optionally, in an embodiment of the present invention, the control device not only controls the pump but also controls the heating device, and controls the heating device and the pump independently of each other.

Optionally, in a further embodiment of the present invention, the control device not only controls the pump but also controls the heating device, and simultaneously controls the heating device and the pump. Preferably, a starting time of the heating device is earlier than a starting time of the pump. Optionally, in the embodiment of the present invention, a time-delay circuit is coupled between the heating device and the pump. The time-delay circuit is used to control the starting time of the heating device to be earlier than the starting time of the pump. Preferably, at least one capacitor is arranged between the heating device and the pump. The heating device is connected in series with the pump. The at least one capacitor is connected in parallel to the pump.

Optionally, in an embodiment of the present invention, the pump is connected in series with a limit switch which is used to limit a travel of the pump. For example, the limit switch is closed under general circumstances. When the pump completely pumps the fluid into the penis implanting mechanism 1, the limit switch is turned off and the pump is stopped.

Optionally, in an embodiment of the present invention, the heating device is arranged on the upper surface inside the reservoir.

Optionally, in an embodiment of the present invention, the penis pacemaker further comprises a temperature sensor, wherein the temperature sensor is coupled to the heating device and used to detect a temperature of the fluid heated by the heating device in real time. The control device controls the heating device to stop heating according to the temperature detected by the temperature sensor. Preferably, the temperature sensor has a threshold. When a temperature detected by the temperature sensor reaches the threshold, the heating device is turned off. The threshold is 34°C to 38°C. Preferably, the threshold is 36.5°C to 37.5°C.

Optionally, in an embodiment of the present invention, a user controls the control device in vitro.

Optionally, in an embodiment of the present invention, the pump delivers the fluid in the reservoir to the penis implanting mechanism via the catheter under the driving of electric energy, and delivers the fluid back to the reservoir via the catheter under the driving of mechanical energy.

Preferably, the pump comprises a pump body, a pump head and a spring. The pump head is connected with the pump body through the spring. The pump head adjusts a volume of the reservoir by means of a reciprocating motion. The spring at least partially drives the pump head to adjust the volume of the reservoir.

Figure 2E is a remotely-controlled heating-type, electric penis pacemaker, comprising a penis implanting mechanism 1, a reservoir 35, a catheter 2, a pump 302, a heating device 36, a power source 32, a control device 702 and a temperature sensor 305. The control device 702 comprises an in-vitro wireless remote-control device 44 and an in-vivo induction device 304. The penis implanting mechanism 1 is arranged between a subcutaneous tissue and a cavernous body of the penis, comprises a hollow cavity and can be axially and elastically fixed at a proximal end of the penis. The reservoir 35 stores a fluid, and is communicated with the cavity of the penis implanting mechanism 1 via the at least one catheter 2. The pump 302 is coupled to the power source 32, the reservoir 35 and the penis implanting mechanism 1. The pump 302 is used to receive electric energy from the power source 32 to deliver the fluid between the reservoir 35 and the penis implanting mechanism 1. The in-vivo induction device 304, the heating device 36 and the temperature sensor are electrically connected to the power source 32 respectively. The temperature sensor is used to detect a temperature of the liquid in the reservoir 35 and transmits a detection result to the in-vitro wireless remote-control device 44. The in-vitro wireless remote-control device 44 controls the heating device 36 to heat according to the detection result. The in-vitro wireless remote-control device 44 comprises a start button which controls the pump and the heating device simultaneously. In addition, the starting time of the heating device is earlier than the starting time of the pump. Optionally, in the embodiment of the present invention, a time-delay circuit is coupled between the heating device and the pump and is used to control the starting time of the heating device to be earlier than the starting time of the pump. Preferably, at least one capacitor is arranged between the heating device and the pump. The heating device is connected in series with the pump. The at least one capacitor is connected in parallel to the pump.

Optionally, in an embodiment of the present invention, the pump is connected in series with a limit switch used to limit a travel of the pump.

The heating device 36 may be arranged inside or outside the reservoir 35. Preferably, the heating device 36 is arranged on the upper surface inside the reservoir 35, such that the fluid is heated by the heating device 36 and is then squeezed by the pump 302 into the catheter 2.

The temperature sensor may be arranged inside or outside the heating device 36. Preferably, the temperature sensor is arranged inside the heating device 36. A main body of the heating device 36 may be in any suitable shape including, but not limited to, a full slab, a plurality of strip plates arranged side by side, a spherical plate, and the like.

The heating device 36 and the pump 302 are started or not started simultaneously. Preferably, the starting time of the heating device 36 is earlier than the starting time of the pump 302. Therefore, the fluid cannot be squeezed by the pump 302 to the catheter 2 before being heated to a specified temperature or temperature range, resulting in a poor experience of the patient. However, if the pump 302 is started after the heating device 36 is started first for heating to an ideal specified temperature or temperature range, such as the temperature for penile erection, the fluid temperature tends to be consistent with the temperature for patient's penis erection, such that the patient will feel uncomfortable.

The ideal specified temperature or temperature range is based on the difference in each patient. The patient may freely decide which temperature is the ideal or suitable temperature.

The temperature sensor has a threshold. When the temperature detected by the temperature sensor reaches the threshold, the heating device 36 is turned off. The threshold is any value or value range between 34 °C and 38 °C. Preferably, the threshold is any value or value range between 36.5 °C and 37.5 °C. In specific implementations, for example, when a temperature detected by the temperature sensor reaches the threshold, a signal is transmitted to the control device 702, and the control device 702 turns off the heating device 36.

Preferably, a valve 22 is further arranged on the catheter 2 and is electrically connected to the power source 32. The valve acquires electric energy from the power source and changes a flowing direction of the fluid under the control of the control device. The valve 22 is an electromagnetic valve or other valve that is controllable by electrical energy.

Optionally, in the embodiment of the present invention, the pump delivers the fluid in the reservoir to the penis implanting mechanism via the catheter under the driving of electric energy, and delivers the fluid back to the reservoir via the catheter under the driving of mechanical energy.

Preferably, the pump comprises a pump body, a pump head and a spring. The pump head is connected with the pump body through the spring. The pump head adjusts a volume of the reservoir by means of a reciprocating motion. The spring at least partially drives the pump head to adjust the volume of the reservoir.

Figure 4 is a schematic diagram of an embodiment of the in-intro wireless remote-control device 44 and the in-vivo induction device 304 based on Figures 2C and 2E.

The in-vitro wireless remote-control device 44 comprises a signal generator and an induction coil. Correspondingly, the in-vivo induction device 304 further comprises an induction coil 701. The induction coil 701 is wirelessly coupled with the induction coil of the in-vitro wireless remote-control device 44 and converts an external energy source into a current signal and transmits it to the control device 702. The control device 702 is used to receive and store an external current signal, and control the power source 32 according to the current signal. The power source 32 is electrically connected with the pump 302 for transmitting electrical energy to the pump 302.

The induction coil may be implanted in any suitable position in the human body. Preferably, the induction coil can be implanted subcutaneously in the lower abdominal wall of the human body. The in-vitro wireless remote-control device 44 sends a wireless signal, such as an electromagnetic wave signal or other type of wireless signal, to the induction coil. The induction coil converts the wireless signal into a current. The current is transmitted to the power source 32 and the control device 702. The control device 702 receives and stores an external signal and program simultaneously and controls the operation of the pump 302, such as starting and turning-off of the pump 302, according to the external signal.

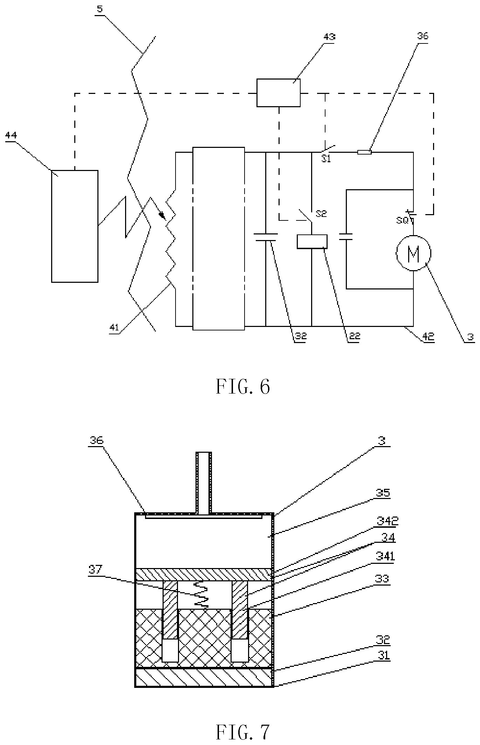

Figure 7 is a schematic diagram of a preferred embodiment of a pump 302 of the penis pacemaker of the present invention based on Figures 2D-2E.

The pump 302 comprises a pump body 33, a pump head 34 and a spring 37. The pump head 34 is connected with the pump body 33 through the spring 37. The pump head 34 adjusts a volume of the reservoir by means of a reciprocating motion. The spring 37 at least partially drives the pump head 34 to adjust the volume of the reservoir.

The pump 302 is composed of a pump body 33 and a pump head 34. A housing 31, a storage battery 32, the pump body 33, the pump head 34, the reservoir 35, and an electric heating module 36 form an integrated structure. The housing 31 is made of a biocompatible material, such as stainless steel, titanium alloy, cobalt chromium alloy, or the like. In addition, the surface of the housing may be sprayed or plated with a material, such as parylene, Teflon, polysaccharide polymer, or modified silicone, for improving the surface properties of the housing and enhancing the biocompatibility and stability. The storage battery 32 may be a lithium ion battery, a lithium-iodine battery, a graphene battery, or the like. The pump body 33 may be powered by the storage battery 32, such that the liquid in the reservoir 35 is pressurized and outputted into the penis implanting mechanism 1, causing an elongation exercise thereof. An electromagnetic valve 22 arranged on the catheter 2 may be powered by the storage battery to implement the on-off control of a liquid path in the catheter 2. The heating device 36 may be powered by the storage battery to change the temperature of the liquid in the reservoir 35, thereby improving the use comfort of the prosthesis.

The pump body 33 may be a motor capable of converting electric energy into a mechanical motion, or a power system consisting of a motor and a transmission mechanism. The motor may be a rotating motor or a linear motor. The transmission mechanism may be a common transmission mechanism such as a gear, a turbine, or a ball screw. In the present invention, it is preferred to select the linear motor as the power system of the pump body. The pump body 33 is used to drive the pump head 34 to move away from the pump body, so that a plunger on the pump head squeezes the liquid in the reservoir 35 to be poured into the penis implanting mechanism 1 through the catheter 2. The pump head 34 is composed of a push rod 341 and a plunger 342. The push rod 341 is driven by the pump body to push the plunger 342 to apply a pressure to the liquid in the reservoir 35, such that the liquid is poured into the penis implanting mechanism 1 through the catheter 2. The plunger 342 and the inner wall of the reservoir 35 can form a sealed cavity. Under the push-pull movement of the plunger 342, the liquid in the reservoir 35 may be pushed out of the reservoir 35 and pumped back to the reservoir 35. Preferably, a tension spring 37 is arranged between the pump head 34 and the pump body 33 in the present invention. That is, the tension spring 37 is stretched when the pump head 34 is pushed by the pump body 33 toward a maximum stroke. In addition, when the pump body 33 is in a non-excited state and the electromagnetic valve on the catheter 2 is in a liquid circuit open state, the pump head 34 in this case is slowly pulled back to an initial position under the tension of the tension spring 37, and meanwhile, the liquid in the penis implanting mechanism 1 is slowly pumped back into the reservoir 35. The process of the pump 302 to pump the liquid outwards is driven by the motor, and the process of the pump 302 to pump the liquid back is acted upon by the tension of the spring, without any other energy consumption, so that the energy efficiency of the storage battery is maximized, thereby reducing the number of times the system is charged.

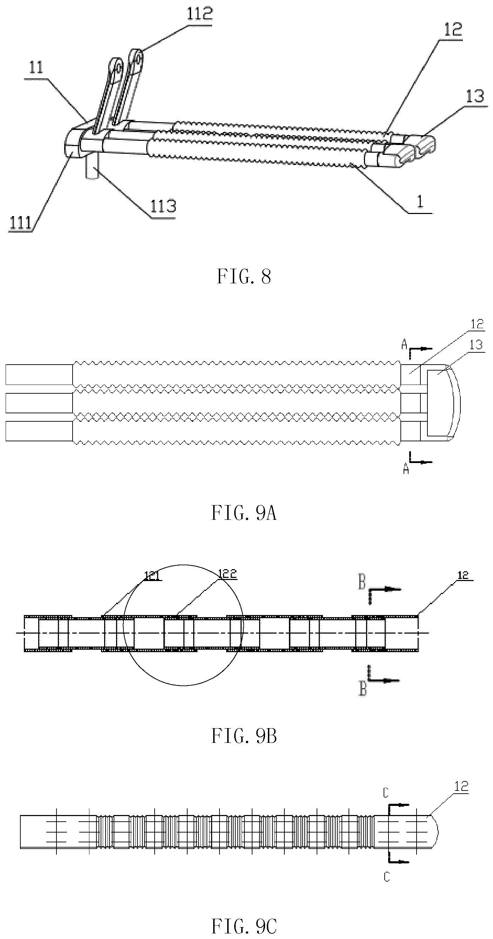

Figures 9A-9D are schematic diagrams of an embodiment of the penis implanting mechanism based on Figures 2A-2E.

The penis implanting mechanism 1 comprises at least two segment-type structures of which the outer surfaces are elongated and retracted along an axial direction of the penis. Preferably, the penis implanting mechanism 1 has a thickness of 0.5mm to 5mm. More preferably, the penis implanting mechanism 1 has a thickness of 2mm to 3mm. The penis implanting mechanism 1 is implanted between a subcutaneous tissue and a cavernous body of the penis. The thickness of the penis implanting mechanism 1 of the present invention is much smaller than the thickness (or radial diameter) of the conventional water bladder of 8 mm to 10 mm, so that it can be implanted in a gap between the subcutaneous layer and the cavernous body of the penis, without destroying the structure of the cavernous body. In addition, the surgical operation is simple, and the physical damage to the patient is minimized.

Preferably, the outer surface of the penis implanting mechanism 1 is provided with a coating layer having biocompatibility and ductility, so that the penis implanting mechanism 1 forms good compatibility and stability with surrounding tissues. The coating layer may be made of a polymer material, such as PTFE, TPU, PU, silica gel, silicone, thermoplastic elastomer, a modified material, or a composite material.

As shown in Figure 9A, the penis implanting mechanism comprises two segment-type structures each including at least two and/or at least three corrugated tubes. Each corrugated structure has a pleated surface in a retracted state, and the surface is stretched to form a smooth surface upon elongation. Preferably, each segment-type structure includes at least three corrugated tubes.

Each corrugated tube is a cylindrical, thin-walled and pleated housing having a plurality of transverse corrugations. The corrugated tube has elasticity and can shift under the action of a pressure, an axial force, a lateral force or a bending moment.

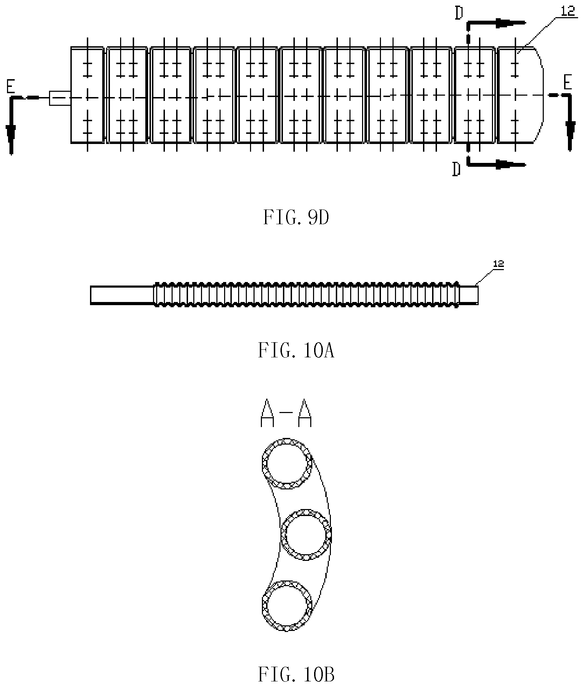

As shown in Figure 10A, preferably, a portion of each segment-type structure close to the distal end of the penis is sealed by an end cover. The shape of the end cover is matched with the cavernous body. Preferably, the end cover comprises a raised upper surface and a recessed lower surface. Preferably, the end covers of the two segment-type structures are coupled with each other to prevent the two segment-type structures from shifting. The coupling manners of the end covers of the two segment-type structures include: the end covers of the two segment-type structures are integrated into a whole, that is, an integral end cover without a gap therebetween; the end covers of the two segment-type structures are connected with each other by welding or the like, so that the joint therebetween is separated by the walls of the two end covers; and other coupling manners applicable to the present invention are available. Preferably, sides of the two segment-type structures are coupled with each other. The coupling manners of the two segment-type structures includes: the two segment-type structures are integrated into a whole from the proximal end to the distal end of the penis; the two segment-type structures are connected with each other by welding or the like; the two segment-type structures and their end covers are integrated into a whole (they can be seen as one segment-type structure in this case), or at least one of the segment-type structures and the end covers thereof is connected with each other by welding or the like; and other coupling manners applicable to the present invention are available.

As shown in Figure 10B, in a preferred embodiment of the present invention, a portion of each segment-type structure close to the distal end of the penis is sealed by an end cover. Corresponding to Figure 9A, every three corrugated tubes form one segment-type structure, and six corrugated tubes form two segment-type structures; a portion of each segment-type structure close to the distal end of the penis is sealed by one end cover, and the portions of every three corrugated tubes close to the distal end of the penis are sealed by one end cover jointly; the end cover is fixed at the distal end of the penis or close to the distal end of the penis; all the corrugated tubes are uniformly distributed between the penis and the cavernous body. The corrugated tubes may be regularly or irregularly distributed between the penis and the cavernous body. The larger the number of corrugated tube structures is, the more uniform the distribution of the penis implanting mechanism 1 between the penis and the cavernous body is, and the better the erection effect is.

Preferably, when each segment-type structure is stretched, the section of the segment-type structure is a closed structure composed of the raised outer surface and the recessed outer surface. The sectional shape of each corrugated structure in a non-expanded state may be a closed crescent shape, a semicircular shape, a circular shape, a quadrangular shape, a circular arc shape, or a quasi-elliptical shape. When each corrugated tube is filled with a liquid having a certain pressure, the corrugated tube can perform an elongational motion in the axial direction.

As shown in Figure 9B, the penis implanting mechanism comprises two segment-type structures. Each segment-type structure comprises at least one outer-layer segment and at least one inner-layer segment, wherein the at least one outer-layer segment and the at least one inner-layer segment are nested with each other and are slidable relative to each other along an axial direction. Preferably, each of the at least one outer-layer segment and the at least one inner-layer segment comprises at least one first hardness segment and at least one second hardness segment, wherein the first hardness segments and the second hardness segments are distributed alternately. The hardness of the first hardness segment is greater than the hardness of the second hardness segment.

Optionally, in an embodiment of the present invention, each segment-type structure comprises one outer-layer segment at each of two ends thereof. Each of the outer-layer segments at two ends comprises a segment unit. Each outer-layer segment and each inner-layer segment between two ends comprise two segment units respectively. Each segment unit comprises two first hardness segments and one second hardness segment, wherein the second hardness segment is positioned between the two first hardness segments. That is, each segment unit comprises a first hardness segment, a second hardness segment, and a first hardness segment that are sequentially connected. Therefore, two segment units of each outer-layer segment and each inner-layer segment are connected and then two first hardness segments are included at the joint therebetween. This design facilitates the interaction of the inner-layer segments and the outer-layer segments.

Preferably, when each segment-type structure is stretched, the section of the segment-type structure is a closed structure composed of a raised outer surface and a recessed outer surface.

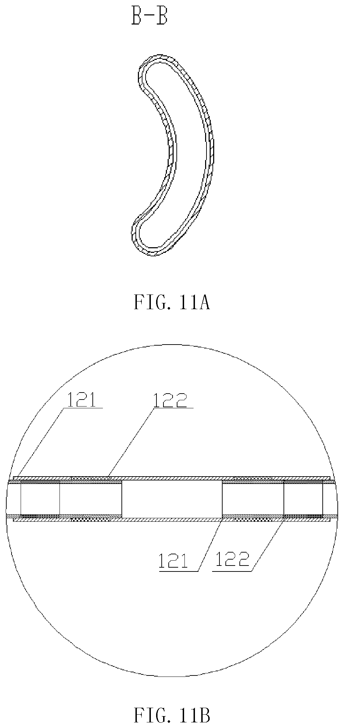

As shown in Figure 11B, the outer layers and the inner layers of the segment-type structures are alternately connected by hard segments 121 and soft segments 122, respectively. When each segment-type structure is filled with a liquid having a certain pressure, soft/hardness segment-type hollow structures that are nested with each other are slidable relative to each other along the axial direction, such that the soft segments of the external soft and hard segment-type hollow structures are staggered from the soft segments of the internal soft and hard segment-type hollow structures mutually, thereby ensuring the rigidity of any axial section.

Preferably, a limiting device is arranged between each outer-layer segment and the corresponding inner-layer segment and used for preventing the outer-layer segment from slipping from the inner-layer segment. The limiting device may be designed such that each outer-layer segment and the corresponding inner-layer segment are connected by a soft material or may be designed in other suitable forms.

Figure 11A is a schematic diagram of a cross-section of the penis implanting mechanism 1 corresponding to Figure 9B. The penis implanting mechanism is a closed structure composed of a raised outer surface and a recessed outer surface. The sectional shape of the closed structure is not limited to the shape shown in Figure 11, and may be other shapes such as a circular shape, a quadrangular shape, a circular arc shape, or a quasi-elliptical shape. Preferably, the cross-section of the segment-type structure when stretched is in a crescent shape.

Preferably, a portion of each segment-type structure close to the distal end of the penis is sealed by an end cover. The shape of the end cover is matched with the shape of the cavernous body. Preferably, the end cover comprises a raised upper surface and a recessed lower surface. Preferably, the end covers of the two segment-type structures are coupled with each other to prevent the two segment-type structures from shifting. The coupling manners of the end covers of the two segment-type structures include: the end covers of the two segment-type structures are integrated into a whole, that is, an integral end cover without a gap therebetween; the end covers of the two segment-type structures are connected with each other by welding or the like, so that the joint therebetween is separated by the walls of the two end covers; and other coupling manners applicable to the present invention are available. Preferably, sides of the two segment-type structures are coupled with each other. The coupling manners of the two segment-type structures includes: the two segment-type structures are integrated into a whole from the proximal end to the distal end of the penis; the two segment-type structures are connected with each other by welding or the like; the two segment-type structures and their end covers are integrated into a whole (they can be seen as one segment-type structure in this case), or at least one of the segment-type structures and the end covers thereof is connected with each other by welding or the like; and other coupling manners applicable to the present invention are available.

Preferably, the penis implanting mechanism 1 has a thickness of 0.5mm to 5mm. More preferably, the penis implanting mechanism 1 has a thickness of 2mm to 3mm.

As shown in Figure 9C, the penis implanting mechanism comprises two segment-type structures. Each segment-type structure comprises at least one hollow variable-length structure and at least one hollow fixed-length structure, wherein the variable-length structure and the fixed-length structure are connected in series with each other. From the outer surface, the variable-length structure and the fixed-length structure are alternately and connected with each other. For convenience of production, the variable-length structure and the fixed-length structure are of a one-time molded structure respectively and made of the same material. The variable-length structure may be a pleated structure or a structure like a corrugated tube. The fixed-length structure is a hollow thin-walled tubular structure. Preferably, when each segment-type structure is stretched, the section of the segment-type structure is a closed structure composed of a raised outer surface and a recessed outer surface. Preferably, the cross-section of the segment-type structure when stretched is in a crescent shape.

Preferably, a supporting structure is arranged between the upper surface and the lower surface of the fixed-length structure and used to support the penis implanting mechanism in a circumferential direction and an axial direction. More preferably, each supporting structure comprises integrated pulling structures, wherein the integrated pulling structures are distributed in the circumferential direction and the axial direction of the segment-type structures.

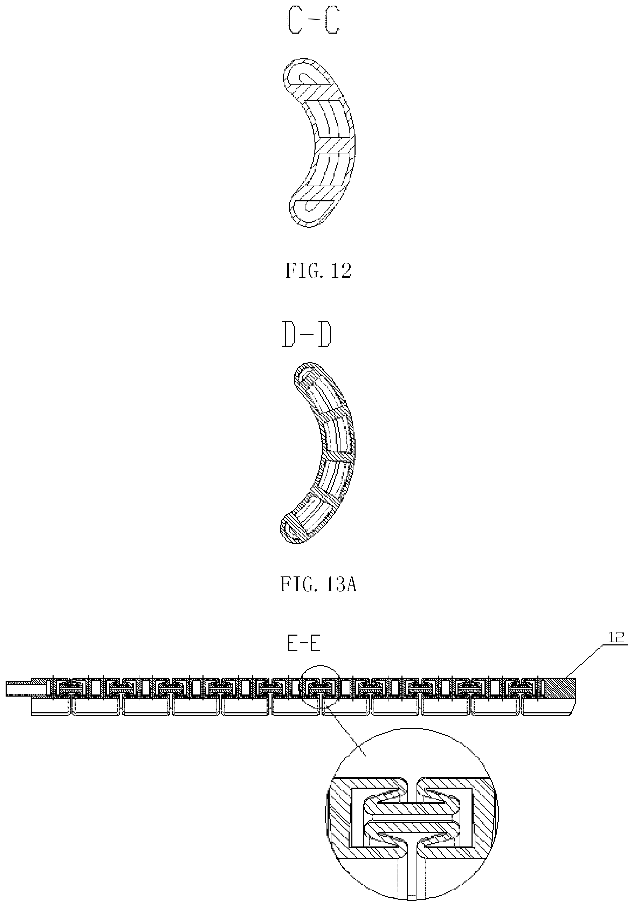

Figure 12 is a schematic diagram of a cross-section of the penis implanting mechanism 1 corresponding to Figure 9C. The penis implanting mechanism 1 is a closed structure composed of a raised outer surface and a recessed outer surface. A supporting structure is arranged between the upper surface and the lower surface of the fixed-length structure. The supporting structure surrounds the inner surface of each segment-type structure by a circle, and the supporting structures which are distributed in the axial direction are connected between the upper surface and the lower surface of the fixed-length structure. The supporting structures in the circumferential direction and the axial direction are integrated. The sectional shape of each segment-type structure is not limited to the shape shown in Figure 12, and may be other shapes such as a circular shape, a quadrangular shape, a circular arc shape, or a quasi-elliptical shape. Preferably, the cross-section of the segment-type structure when stretched is in a crescent shape.

Preferably, a portion of each segment-type structure close to the distal end of the penis is sealed by an end cover. The shape of the end cover is matched with the shape of the cavernous body. Preferably, the end cover comprises a raised upper surface and a recessed lower surface. Preferably, the end covers of the two segment-type structures are coupled with each other to prevent the two segment-type structures from shifting. The coupling manners of the end covers of the two segment-type structures include: the end covers of the two segment-type structures are integrated into a whole, that is, an integral end cover without a gap therebetween; the end covers of the two segment-type structures are connected with each other by welding or the like, so that the joint therebetween is separated by the walls of the two end covers; and other coupling manners applicable to the present invention are available. Preferably, sides of the two segment-type structures are coupled with each other. The coupling manners of the two segment-type structures includes: the two segment-type structures are integrated into a whole from the proximal end to the distal end of the penis (they can be seen as one segment-type structure in this case); the two segment-type structures are connected with each other by welding or the like; the two segment-type structures and their end covers are integrated into a whole, or at least one of the segment-type structure and the end cover thereof is connected with each other by welding or the like; and other coupling manners applicable to the present invention are available.

Preferably, the penis implanting mechanism 1 has a thickness of 0.5mm to 5mm. More preferably, the penis implanting mechanism 1 has a thickness of 2mm to 3mm.

As shown in Figure 9D, the penis implanting mechanism comprises two segment-type structures. Each segment-type structure comprises at least one flexible foldable structure. A folded portion of the flexible foldable structure is stretched during fluid injection to cause the penis implanting mechanism to elongate. When the fluid is discharged, the flexible foldable structure is folded into a predetermined shape by its own elastic force to cause the penile implant mechanism to retract. Preferably, when each segment-type structure is stretched, the section of the segment-type structure is a closed structure composed of a raised outer surface and a recessed outer surface. Preferably, the cross-section of the segment-type structure when stretched is in a crescent shape.

Preferably, a supporting structure is respectively arranged between every two of the flexible foldable structures and used to support the penis implanting mechanism in a circumferential direction and an axial direction. Preferably, each supporting structure comprises an integrated pulling structure, wherein the integrated pulling structures are distributed in the circumferential direction and the axial direction of the segment-type structures.

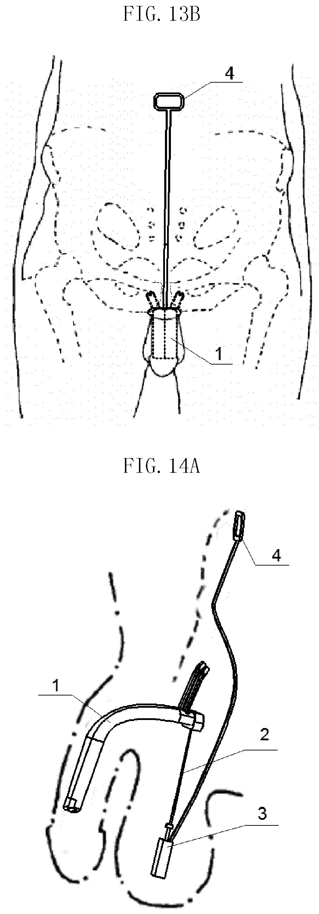

As shown in Figure 13A, each segment-type structure has a one-piece outer surface. The outer surface comprises a plurality of flexible foldable structures, and a supporting structure is arranged between every two of the flexible foldable structures. The folded portion is stretched when the fluid is injected into the segment-type structure, and is folded into a predetermined shape, for example, a folded structure as shown in Figure 13B, under the action of its own elastic force when the fluid is discharged. Every two of the foldable structures are separated by the supporting structure. The folded structure shown in Fig. 13B is only used to illustrate the configuration of the folded structure, and the folded structure of the present invention is not limited to the configuration shown in Fig. 13B.

Preferably, a portion of the segment-type structure close to the distal end of the penis is sealed by an end cover. The shape of the end cover is matched with the shape of the cavernous body. Preferably, the end cover comprises a raised upper surface and a recessed lower surface. Preferably, the end covers of the two segment-type structures are coupled with each other to prevent the two segment-type structures from shifting. The coupling manners of the end covers of the two segment-type structures include: the end covers of the two segment-type structures are integrated into a whole, that is, an integral end cover without a gap therebetween; the end covers of the two segment-type structures are connected with each other by welding or the like, so that the joint therebetween is separated by the walls of the two end covers; and other coupling manners applicable to the present invention are available. Preferably, sides of the two segment-type structures are coupled with each other. The coupling manners of the two segment-type structures includes: the two segment-type structures are integrated into a whole from the proximal end to the distal end of the penis (they can be seen as one segment-type structure in this case); the two segment-type structures are connected to each other by welding or the like; the two segment-type structures and their end covers are integrated into a whole, or at least one of the segment-type structure and the end cover thereof is connected with each other by welding or the like; and other coupling manners applicable to the present invention are available.

The segment-type structure of the present invention can be printed by a 3D printer using a suitable material.

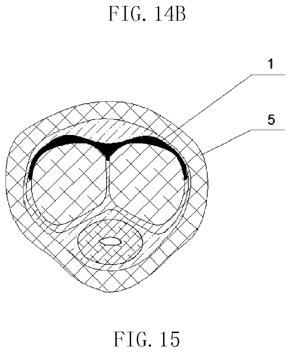

As shown in Figure 15, the penis implanting mechanism is arranged between a subcutaneous tissue and a cavernous body of the penis.

Figure 8 is a schematic diagram of an embodiment in which the penis implanting mechanism is fixed to the proximal end of the penis based on Figures 2A-2E.

In this embodiment, the penis implanting mechanism 1 is provided with a fixing device at the proximal end of the penis. The fixing device is coupled to the pubis. As shown in Figure 8, optionally, in this embodiment of the present invention, the fixing device is at least one fixing plate provided with a through hole through which a fixing member fixes the fixing plate to the pubis. The fixing member is a medical screw, a fastener or the like. Preferably, the fixing device is two fixing plates each provided with a through hole. The shape of the fixing plate is not limited. Although the fixing plate is fixed to the pubis here, it can also be fixed to other positions of the human body.

As shown in Figure 8, a base is arranged at the proximal end of the penis implanting mechanism. The fixing device is coupled to the base. The base is provided with a hollow cavity which is communicated with the cavity of the penis implanting mechanism. The proximal end of the segment-type structure is connected to the base. The base is communicated with the interior of the segment-type structure. When the pump 302 is started, the fluid is first outputted to the base and is then evenly distributed throughout the one or more segment-type structures via the base. The base functions to secure the penis implanting mechanism 1 and uniformly distribute the fluid at the same time.

One base may be available, and as shown in Figure 8, it is further provided with two protruding interfaces which are respectively coupled with each segment-type structure respectively. Preferably, the shape of the base is matched with the shape of the cavernous body, thereby preventing the patient feeling uncomfortable because the shape of the base is unmatched with the shape of the cavernous body. Preferably, the base comprises a raised upper surface and a recessed lower surface, such that an outer profile of the base is matched with an outer profile of the segment-type structure, and accordingly the penis implanting mechanism 1 and the penis are consistent in appearance and the patient feels more comfortable. Two bases are also available, and each base is coupled with each segment-type structure.

Preferably, a portion of each segment-type structure close to the distal end of the penis is sealed by an end cover. The end shape of the end cover is matched with the shape of the cavernous body.

Preferably, each end cover comprises a raised upper surface and a recessed lower surface, such that the outer profile of the end cover is matched with the outer profile of the base and/or the segment-type structure, and therefore, the patient feels more comfortable.

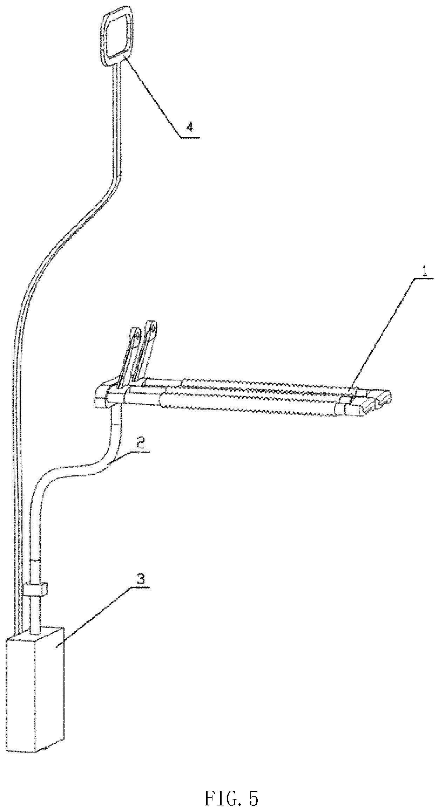

Figure 5 is a schematic diagram of a preferred embodiment of the penis pacemaker based on Figure 2E. Figure 5 is a remotely-controlled heating-type, electric penis pacemaker, comprising a penis implanting mechanism 1, a reservoir 25, a catheter 2, a pump 302, a power source 32, a control device 702, a heating device, a temperature sensor, an in-vitro wireless remote-control device 44 and an in-vivo induction device 304. A portion of the penis implanting mechanism 1 close to the proximal end of the penis is provided with a base 11, and a portion of the penis implanting mechanism 1 close to the distal end of the penis is provided with an end cover 13.

As shown in Figures 14A-14B, the subcutaneous penis implanting mechanism 1 is implanted between a subcutaneous tissue and a cavernous body of the penis and is connected with the pump 302 through the catheter 2. The pump 302 is placed in the scrotum. A fluid can be injected to or drained from the subcutaneous penis implanting mechanism by starting and stopping the pump 302, thereby achieving two physiological states of erection and weakness of the penis.

A main body 111 of the base is a cavity-type structure for communicating the catheter 2 and the penis implanting mechanism 1. The base 11 is further provided with a penis fixing plate 112 which is used to secure the entire base on the pubis, so as to provide a fulcrum force when the penis implanting mechanism 1 performs an elongational motion, thereby ensuring that the change in the length of the penis implanting mechanism 1 is an elongational motion along the base 11 towards the end cover 13.

The catheter 2 is a hollow liquid catheter of which a wall lining is of a mesh-like woven structure that increases strength. One end of the catheter 2 is connected to the pump 302, and the other end of the catheter 2 is connected to the base 11, for communicating a liquid path therebetween. The catheter 2 is provided with an electromagnetic valve 22 that can control the liquid path to be opened or closed.

The reservoir 35, the pump 302, the power source 32, the control device 702, the heating device and the temperature sensor form an integrated structure which is surrounded by a housing 31. The heating device 36 is arranged on the inner surface of the reservoir 35 and used to heat the liquid in the reservoir 35. The temperature sensor which can control a temperature of the liquid in the reservoir 35 accurately is arranged inside the heating device. The housing 31 is made of a biocompatible material, such as stainless steel, titanium alloy, cobalt chromium alloy, or the like. In addition, the surface of the housing may be sprayed or plated with a material, such as parylene, Teflon, polysaccharide polymer, or modified silicone, for improving the surface properties of the housing and enhancing the biocompatibility and stability. The battery 32 is a storage battery 32. The pump body 33 of the pump 302 may be powered by the storage battery 32, such that the liquid in the reservoir 35 is pressurized and outputted into the penis implanting mechanism 1, causing an elongation exercise thereof. An electromagnetic valve 22 arranged on the catheter 2 may be powered by the storage battery to implement the on-off control of the liquid path in the catheter 2. The heating device 36 may be powered by the storage battery to change the temperature of the liquid in the reservoir 35, thereby improving the use comfort of the prosthesis.

The in-vitro wireless remote-control device 44 comprises a signal generator and an induction coil. Correspondingly, the in-vivo induction device 304 further comprises an induction coil 701. The induction coil 701 is wirelessly coupled with the induction coil of the in-vitro wireless remote-control device 44 and converts an external energy source into a current signal and transmits it to the control device 702. The control device 702 is used to receive and store an external current signal, and control the power source 32 according to the current signal. The power source 32 is electrically connected with the pump 302 for transmitting electrical energy to the pump 302.

The induction coil may be implanted in any suitable position in the human body. Preferably, the induction coil may be implanted subcutaneously in the lower abdominal wall of the human body. The in-vitro wireless remote-control device 44 sends a wireless signal, such as an electromagnetic wave signal or other type of wireless signal, to the induction coil. The induction coil converts the wireless signal into a current. The current is transmitted to the power source 32 and the control device 702. The control device 702 receives and stores an external signal and program simultaneously and controls the operations of the pump 302, the electromagnetic valve 22 and the heating device 36, according to the external signal.

Figure 6 is a schematic diagram of an embodiment of a control circuit of the penis pacemaker of the present invention based on Figure 5.

At least one capacitor is arranged between the heating device and the pump. The heating device is connected in series with the pump. The at least one capacitor is connected in parallel to the pump. The pump is connected in series with the limit switch which is used to control a travel of the pump.

The storage battery 32 is wirelessly coupled with the in-vitro wireless remote-control device 44 through an induction coil 41 to complete charging, and supplies electric energy to the pump 302, the heating device 36 and the electromagnetic valve 22. During the erection, the control device 702 controls a switch S1 to be turned off; the heating device 36 heats the liquid in the reservoir 35, the capacitor starts to be charged, and the pump 302 is shortcircuited and does not work. After the charging of the capacitor is finished, the pump 302 is started to inject the heated liquid in the reservoir 35 into the penis implanting mechanism 1 through the catheter 2, and the electromagnetic valve is automatically turned on under the impact of the liquid. During the weakness, the control device 702 controls the switch S1 to be turned off, S2 is turned on, and the electromagnetic valve 22 is electrified. The liquid in this case flows back to the reservoir 35 under the action of the spring 37 between the pump head 34 and the pump body 33 to complete liquid drainage of the penis implanting mechanism 1.

Correspondingly, the present invention further provides a method for simulating penis erection and weakness by using the penis pacemaker of the present invention. The method comprises the following steps:

driving, by the pump 302, the delivery of the fluid from the reservoir 35 to the cavity of the penis implanting mechanism 1 via the catheter 2, such that the penis implanting mechanism 1 is axially elongated to cause the penis erection; and

driving, by the pump 302, the delivery of the fluid from the cavity of the penis implanting mechanism 1 to the reservoir 35 via the catheter 2, such that the penis implanting mechanism 1 is axially retracted to cause the penis weakness.

Correspondingly, the present invention further provides a method for simulating penis erection and weakness by using the penis pacemaker of the present invention. The method comprises the following steps:

driving, by the pump 302, the delivery of the fluid from the reservoir 35 to the cavity of the penis implanting mechanism 1 via the catheter, such that the penis implanting mechanism 1 is axially elongated to cause the penis erection, wherein the heating device 36 heats the fluid outputted from the reservoir 35 to the penis implanting mechanism 1; and

driving, by the pump 302, the delivery of the fluid from the cavity of the penis implanting mechanism 1 to the reservoir 35 via the catheter, such that the penis implanting mechanism 1 is axially retracted to cause the penis weakness.

The above methods can be implemented by executing the device provided by the embodiments of the present invention, involve the corresponding functional modules for implementing corresponding functions of the device, and achieve the corresponding beneficial effects. For technical details that are not described in detail in this embodiment, references may be made to the device provided by the embodiments of the present invention.

In summary, the traditional penis implanting mechanism consists of two water bladders which are directly implanted into the two cavernous bodies, but the implantation cannot be completed until the cavernous body tissues are completely dissociated respectively during a surgical procedure, such that the anatomical structure of the cavernous tissues themselves is destroyed and a patient may even permanently lose the possibility of recovery. However, the penis implanting mechanism in the embodiments of the present invention is arranged between the subcutaneous tissue and the cavernous body of the penis, and has a thickness far smaller than the diameter or the thickness of the traditional water bladder, thereby hardly damaging the anatomical structure of the cavernous body tissues.

The ends of the water bladders of the traditional penis implanting mechanism are inserted into the body through two tapered structures. However, the penis implanting mechanism in the embodiments of the present invention is implanted between the subcutaneous tissue and the cavernous body of the penis, is thus not suitable for being inserted into the body in a tapered structure, instead being fixed to the pubis by a fixing device such as a fixing plate. Therefore, the penis implanting mechanism can be fixed more firmly, and is not prone to shifting.

Further, in the embodiments of the present invention, the outer surface of the penis implanting mechanism is designed as segment-type structures that are elongated and retracted along the axial direction of the penis, for example, in a manner of a plurality of corrugated tube structures, mutual nesting of segment-type hollow structures of different hardness, and mutually serial connection of variable-length structures and fixed-length structures. Therefore, when the penis is in an erection, the penis implanting mechanism can be elongated, which causes the penis to elongate. When the penis is weak, the penile implanting mechanism retracts to a length consistent with the length of the penis.

In addition, the hollow base is arranged at the proximal end of each segment-type structure, and the cavity of the base is communicated with the cavity of the segment-type structure, such that the segment-type structure can be firmly fixed between the subcutaneous tissue and the cavernous body of the penis by the base, without shifting. On the other hand, when a plurality of segment-type structures is arranged, after the pump is started, the fluid is first outputted to the base and then evenly distributed in the segment-type structures via the bases. The bases simultaneously function to secure the penis implanting mechanism and distribute the fluid evenly.

In an embodiment of the present invention, the heating device is additionally arranged in the penis pacemaker to heat the fluid outputted from the reservoir to the penis implanting mechanism, thereby eliminating the temperature difference between the fluid in the reservoir and the penis part, such that a patient feels more comfortable.

Furthermore, the temperature sensor is further additionally arranged in the penis pacemaker. The heating device is powered by the power source. The control device controls the heating device and the temperature sensor. The temperature sensor has a threshold. After the temperature of the fluid reaches the threshold, a signal is transmitted to the control device. The control device then controls the pump to deliver the fluid from the reservoir to the penis implanting mechanism, thereby effectively controlling the heating temperature of the fluid.

In a further embodiment of the present invention, the heating device and the temperature sensor are controlled in a remotely controlled manner, such that the patient's operation is more convenient. Further, the starting time of the heating device is caused to be earlier than the starting time of the pump by means of connecting the capacitor in parallel with the pump, arranging the capacitor between the heating device and the pump, etc. Therefore, the fluid in the reservoir is prevented from flowing into the penis implanting mechanism without being heated. Meanwhile, the structure of a remote-controller can also be simplified, for example, buttons that control the heating device and the pump can be combined into one.

The above-described embodiments are merely illustrative of several embodiments of the present invention, and the description thereof is more specific and detailed, but is not to be construed as limiting the scope of the invention. It should be noted that a number of variations and modifications may be made by those ordinary skilled in the art without departing from the concept of the present invention, and all of them fall into the scope of protection the present invention. Therefore, the scope of protection of the present invention should be subjected to the appended claims.

## Claims

1. A penis pacemaker, comprising a penis implanting mechanism, a reservoir, a catheter and a pump, wherein,
the penis implanting mechanism is arranged between a subcutaneous tissue and a cavernous body of the penis, comprises a hollow cavity and can be axially and elastically fixed at a proximal end of the penis;
the reservoir stores a fluid, and is communicated with the cavity of the penis implanting mechanism via the at least one catheter; and
the pump is coupled to the reservoir and the penis implanting mechanism, and used to provide power for the delivery of the fluid between the reservoir and the penis implanting mechanism.

2. The penis pacemaker of claim 1, further comprising a heating device, wherein the heating device is coupled to the reservoir and used to heat the fluid outputted from the reservoir to the penis implanting mechanism.

3. The penis pacemaker of claim 1, further comprising a power source, wherein the power source is electrically connected to the pump and used to supply electric energy to the pump.

4. The penis pacemaker of claim 2, further comprising a power source, wherein the power source is electrically connected to the pump and/or the heating device and used to supply electric energy to the pump and/or the heating device.

5. The penis pacemaker of claim 1, wherein the pump comprises a pump body, a pump head and a spring, wherein the pump head is connected with the pump body through the spring; the pump head adjusts a volume of the reservoir by means of a reciprocating motion; and the spring at least partially drives the pump head to adjust the volume of the reservoir.

6. The penis pacemaker of claim 1, further comprising a control device, wherein the control device is used to control the operation of the pump.

7. The penis pacemaker of claim 3, further comprising a control device, wherein the control device is used to control the operation of the pump and/or the heating device.

8. The penis pacemaker of claim 7, wherein the control device comprises a limit switch, wherein the limit switch is connected in series with the pump and used to limit a travel of the pump.

9. The penis pacemaker of claim 2, wherein a time-delay circuit is coupled between the heating device and the pump, wherein the time-delay circuit is used to control a starting time of the heating device to be earlier than a starting time of the pump.

10. The penis pacemaker of claim 9, wherein at least one capacitor is arranged between the heating device and the pump; the heating device is connected in series with the pump; and the at least one capacitor is connected in parallel to the pump.

11. The penis pacemaker of claim 2, wherein the heating device is arranged on an upper surface inside the reservoir.

12. The penis pacemaker of claim 2, further comprising a temperature sensor, wherein the temperature sensor is coupled to the heating device and used to detect a temperature of the fluid heated by the heating device in real time; and the control device controls the heating device to stop heating according to the temperature detected by the temperature sensor.

13. The penis pacemaker of claim 12, wherein the temperature sensor has a threshold; when a temperature detected by the temperature sensor reaches the threshold, the heating device is turned off; and the threshold is 34°C to 38°C.

14. The penis pacemaker of claim 6, wherein the control device comprises an in-vitro wireless remote-control device and an in-vivo induction device, wherein the in-vivo induction device is used to be wirelessly coupled to the in-vitro wireless remote-control device and charge the power source according to a coupling signal.

15. The penis pacemaker of claim 7, wherein the control device comprises an in-vitro wireless remote-control device and an in-vivo induction device, wherein the in-vivo induction device is used to be wirelessly coupled to the in-vitro wireless remote-control device and charge the power source according to a coupling signal.

16. The penis pacemaker of claim 15, wherein the in-vitro wireless remote-control device comprises a start button, wherein the start button is used to control the starting of the pump and/or the starting of the heating device.

17. The penis pacemaker of claim 7, wherein at least one valve is further arranged on the catheter, wherein the valve acquires electrical energy from the power source and changes a flowing direction of the fluid under the control of the control device.

18. The penis pacemaker of claim 1, wherein a fixing device is arranged at a proximal end of the penis implementing mechanism and coupled to a pubis.

19. The penis pacemaker of claim 1, wherein the penis implementing mechanism comprises at least two segment-type structures, outer surfaces whereof can be axially elongated and retracted along an axial direction of the penis.

20. The penis pacemaker of claim 19, wherein each segment-type structure comprises at least two and/or at least three corrugated tubes.

21. The penis pacemaker of claim 19, wherein each segment-type structure comprises at least one outer-layer segment and at least one inner-layer segment, wherein the at least one outer-layer segment and the at least one inner-layer segment are nested with each other and are slidable relative to each other along an axial direction.

22. The penis pacemaker of claim 21, wherein a limiting device is arranged between each outer-layer segment and the corresponding inner-layer segment and used for preventing the outer-layer segment from slipping from the inner-layer segment.

23. The penis pacemaker of claim 21, wherein each of the at least one outer-layer segment and the at least one inner-layer segment comprises at least one first hardness segment and at least one second hardness segment, wherein the first hardness segments and the second hardness segments are distributed alternately; and the hardness of the first hardness segment is greater than the hardness of the second hardness segment.

24. The penis pacemaker of claim 23, wherein each segment-type structure comprises one outer-layer segment at each of two ends thereof, wherein each of the outer-layer segments at two ends comprises a segment unit; each outer-layer segment and each inner-layer segment between two ends comprise two segment units respectively; and each segment unit comprises two first hardness segments and one second hardness segment, wherein the second hardness segment is positioned between the two first hardness segments.

25. The penis pacemaker of claim 19, wherein each segment-type structure comprises at least one hollow variable-length structure and at least one hollow fixed-length structure, wherein the variable-length structure and the fixed-length structure are connected in series with each other.

26. The penis pacemaker of claim 25, wherein a supporting structure is arranged between an upper surface and a lower surface of each fixed-length structure and used for supporting the penis implanting mechanism in a circumferential direction and an axial direction.

27. The penis pacemaker of claim 19, wherein each segment-type structure comprises at least one flexible foldable structure; a folded portion of the flexible foldable structure is stretched during fluid injection to cause the penis implanting mechanism to elongate; and when the fluid is discharged, the flexible foldable structure is folded into a predetermined shape by its own elastic force to cause the penile implant mechanism to retract.

28. The penis pacemaker of claim 27, wherein a supporting structure is respectively arranged between every two of the flexible foldable structures and used to support the penis implanting mechanism in a circumferential direction and an axial direction.

29. The penis pacemaker of claim 26 or 28, wherein each supporting structure comprises an integrated pulling structure, wherein the integrated pulling structures are distributed in the circumferential direction and the axial direction of the segment-type structures.

30. The penis pacemaker of claim 19, wherein a portion of each segment-type structure close to the distal end of the penis is sealed by an end cover, wherein the shape of the end cover is matched with that of the cavernous body.

31. The penis pacemaker of claim 30, wherein each end cover comprises a raised upper surface and a recessed lower surface.

32. The penis pacemaker of claim 30, wherein the penis implanting mechanism comprises two segment-type structures, wherein the end covers of the two segment-type structures are coupled with each other.

33. The penis pacemaker of claim 18, wherein a base is arranged at a proximal end of the penis implanting mechanism; the fixing device is coupled to the base; and the base is provided with a hollow cavity which is communicated with the cavity of the penis implanting mechanism.

## Amended claims

### Amended claims under Art. 19.1 PCT

1. A penis pacemaker, comprising a penis implanting mechanism, a reservoir, a catheter, a pump and a power source, wherein,
the penis implanting mechanism is arranged between a subcutaneous tissue and a cavernous body of the penis, comprises a hollow cavity and can be axially and elastically fixed at a proximal end of the penis;
the reservoir stores a fluid, and is communicated with the cavity of the penis implanting mechanism via the at least one catheter;
the pump is coupled to the reservoir and the penis implanting mechanism, and used to provide power for the delivery of the fluid between the reservoir and the penis implanting mechanism; and
the power source is electrically connected to the pump and used to supply electric energy to the pump.

2. The penis pacemaker of claim 1, further comprising a heating device, wherein the heating device is coupled to the reservoir and used to heat the fluid outputted from the reservoir to the penis implanting mechanism; and the power source is electrically connected to the heating device and used to supply electric energy to the heating device.

3. The penis pacemaker of claim 1, wherein the pump comprises a pump body, a pump head and a spring, wherein the pump head is connected with the pump body through the spring; the pump head adjusts a volume of the reservoir by means of a reciprocating motion; and the spring at least partially drives the pump head to adjust the volume of the reservoir.

4. The penis pacemaker of claim 2, wherein a time-delay circuit is coupled between the heating device and the pump, wherein the time-delay circuit is used to control a starting time of the heating device to be earlier than a starting time of the pump.

5. The penis pacemaker of claim 4, wherein at least one capacitor is arranged between the heating device and the pump; the heating device is connected in series with the pump; and the at least one capacitor is connected in parallel to the pump.

6. The penis pacemaker of claim 1, further comprising a control device, wherein the control device is used to control the operation of the pump.

7. The penis pacemaker of claim 6, wherein the control device comprises a limit switch, wherein the limit switch is connected in series with the pump and used to limit a travel of the pump.

8. The penis pacemaker of claim 2, further comprising a temperature sensor, wherein the temperature sensor is coupled to the heating device and used to detect a temperature of the fluid heated by the heating device in real time; the temperature sensor has a threshold; when a temperature detected by the temperature sensor reaches the threshold, the heating device is turned off; the threshold is 34°C to 38°C; and the control device controls the heating device to stop heating according to the temperature detected by the temperature sensor.

9. The penis pacemaker of claim 6, wherein the control device comprises an in-vitro wireless remote-control device and an in-vivo induction device, wherein the in-vivo induction device is used to be wirelessly coupled to the in-vitro wireless remote-control device and charge the power source according to a coupling signal.

10. The penis pacemaker of claim 1, wherein the proximal end of the penis implanting mechanism is provided with:
a fixing device, which is coupled to a pubis and is at least one fixing plate provided with a through hole through which a fixing member fixes the fixing plate to the pubis; and
a base, to which the fixing device is coupled and which is provided with a hollow cavity communicated with the cavity of the penis implanting mechanism, wherein the shape of the base is matched with the shape of the cavernous body.

11. The penis pacemaker of claim 1, wherein the penis implementing mechanism comprises at least two segment-type structures of which outer surfaces can be elongated and retracted along an axial direction of the penis.

12. The penis pacemaker of claim 11, wherein each segment-type structure comprises at least two and/or at least three corrugated tubes.

13. The penis pacemaker of claim 11, wherein each segment-type structure comprises at least one outer-layer segment and at least one inner-layer segment, wherein the at least one outer-layer segment and the at least one inner-layer segment are nested with each other and are slidable relative to each other along an axial direction.

14. The penis pacemaker of claim 13, wherein a limiting device is arranged between each outer-layer segment and the corresponding inner-layer segment and used for preventing the outer-layer segment from slipping from the inner-layer segment.

15. The penis pacemaker of claim 13, wherein each of the at least one outer-layer segment and the at least one inner-layer segment comprises at least one first hardness segment and at least one second hardness segment, wherein the first hardness segments and the second hardness segments are distributed alternately; and the hardness of the first hardness segment is greater than the hardness of the second hardness segment.

16. The penis pacemaker of claim 15, wherein each segment-type structure comprises one outer-layer segment at each of two ends thereof, wherein each of the outer-layer segments at two ends comprises a segment unit; each outer-layer segment and each inner-layer segment between two ends comprise two segment units respectively; and each segment unit comprises two first hardness segments and one second hardness segment, wherein the second hardness segment is positioned between the two first hardness segments.

17. The penis pacemaker of claim 11, wherein each segment-type structure comprises at least one hollow variable-length structure and at least one hollow fixed-length structure, wherein the variable-length structure and the fixed-length structure are connected in series with each other.

18. The penis pacemaker of claim 17, wherein a supporting structure is arranged between an upper surface and a lower surface of the fixed-length structure and used for supporting the penis implanting mechanism in a circumferential direction and an axial direction.

19. The penis pacemaker of claim 11, wherein each segment-type structure comprises at least one flexible foldable structure; a folded portion of each flexible foldable structure is stretched during fluid injection to cause the penis implanting mechanism to elongate; and when the fluid is discharged, the flexible foldable structure is folded into a predetermined shape by its own elastic force to cause the penile implant mechanism to retract.

20. The penis pacemaker of claim 19, wherein a supporting structure is respectively arranged between every two of the flexible foldable structures and used to support the penis implanting mechanism in a circumferential direction and an axial direction.

21. The penis pacemaker of claim 19 or 20, wherein each supporting structure comprises an integrated pulling structure, wherein the integrated pulling structures are distributed in the circumferential direction and the axial direction of the segment-type structures.

22. The penis pacemaker of claim 11, wherein the penis implanting mechanism comprises two segment-type structures, wherein the end covers of the two segment-type structures are coupled with each other.

23. The penis pacemaker of claim 1, wherein the penis implanting mechanism has a thickness of 0.5mm to 5mm.
